# EUROPÄISCHE PATENTSCHRIFT

(11) **EP 1 968 538 B1**
(45) Veröffentlichungstag und Bekanntmachung des Hinweises auf die Patenterteilung: **25.04.2012**
(21) Anmeldenummer: 06818907.5
(22) Anmeldetag: 29.11.2006
(51) Int. Cl.: A61K 9/00, A61K 9/127, A61K 38/13

(54) **PHARMAZEUTISCHE ZUSAMMENSETZUNGEN MIT CYCLOSPORIN**
PHARMACEUTICAL COMPOSITIONS COMPRISING CYCLOSPORIN
COMPOSITIONS PHARMACEUTIQUES A BASE DE CYCLOSPORINE

(30) Priorität: 06.12.2005 DE 102005058252; 31.10.2006 DE 102006051512
(43) Veröffentlichungstag der Anmeldung: 17.09.2008
(73) Patentinhaber: PARI Pharma GmbH, 82319 Starnberg (DE)
(72) Erfinder: KELLER, Manfred, 81379 Munchen (DE); AKKAR, Aslihan, 80802 München (DE); Mehrwald, Ralf, 80687 München (DE)
(74) Vertreter: Beckmann, Claus
(86) Internationale Anmeldenummer: PCT/EP2006/011459
(87) Internationale Veröffentlichungsnummer: WO 2007/065588

(56) Entgegenhaltungen:
- EP-A- 1 712 220
- WO-A-00/40219
- WO-A2-2005/037246
- US-A- 5 660 858
- US-A1- 2002 013 271

## Beschreibung

### TECHNISCHES GEBIET DER ERFINDUNG

Die Erfindung betrifft flüssige pharmazeutische Zubereitungen, die als Wirkstoff Cyclosporin enthalten sowie Substanzen mit ähnlichen chemisch physikalischen und therapeutischen Eigenschaften und die für die orale, parenterale, nasale, okulare, mukosale, topische und insbesondere für die pulmonale Anwendung geeignet sind. In weiteren Aspekten werden Behältnisse zur Verpackung und Anwendung der Zubereitungen und Konzentrate davon beschrieben. Die Erfindung betrifft ferner die pharmazeutischen Verwendungen der Zubereitungen und ihren Einsatz zur Behandlung bestimmter Erkrankungen.

### HINTERGRUND DER ERFINDUNG

Cyclosporin (auch Ciclosporin) ist ein zyklisches Oligopeptid mit immunsuppressiver und calcineurinhemmender Wirkung. Es zeichnet sich durch einen selektiven und reversiblen Mechanismus der Immunsuppression aus. Es blockiert selektiv die Aktivierung von T-Lymphozyten über die Produktion von bestimmten Zytokinen, die an der Regulierung dieser T-Zellen beteiligt sind. Dabei wird vor allem die Synthese von Interleukin-2 gehemmt, wodurch gleichzeitig die Proliferation von zytotoxischen T-Lymphozyten, die z. B. für die Abstoßung von fremdem Gewebe verantwortlich sind, supprimiert wird. Cyclosporin wirkt intrazellulär durch Bindung an die so genannten Cyclophiline oder Immunophiline, die zur Familie der Cyclosparin-bindenden Proteine mit hoher Affinität gehören. Der Komplex aus Cyclosporin und Cyclophilin blockiert anschließend die Serin-Threonin-Phosphatase Calcineurin. Deren Aktivitätszustand steuert wiederum die Aktivierung von Transkriptionsfaktoren wie etwa NF-KappaB oder NFATp/c, welche bei der Aktivierung verschiedener Zytokin-Gene, darunter auch das Interleukin-2, eine entscheidende Rolle spielen. Hierdurch werden die immunkompetenten Lymphozyten während der G0- oder G1-Phase des Zellzyklus arretiert, da die für die Zellteilung essentiellen Proteine wie Interleukin-2 nicht mehr produziert werden können. T-Helfer-Zellen, welche die Aktivität der für die Abstoßung verantwortlichen zytotoxischen T-Zellen erhöhen, sind der bevorzugte Angriffspunkt für Cyclosporin.

Daneben inhibiert Cyclosporin die Synthese und Freisetzung weiterer Lymphokine, die für die Proliferation reifer zytotoxischer T-Lymphozyten und für weitere Funktionen der Lymphozyten zuständig sind. Die Fähigkeit von Cyclosporin, Interleukin-2 zu blockieren, ist für seine klinischer Wirksamkeit maßgeblich: Transplantatempfänger, die ihre Transplantate gut tolerierten, zeichnen sich durch eine niedrige Produktion von Interteukin-2 aus. Dagegen ist bei Patienten mit manifester Abstoßungsreaktion keine Hemmung der Interleukin-2-Produktion feststellbar.

Als erstes und bisher einziges Cyclosporin kam Cyclosporin A in den 80er Jahren auf den Markt. Chemisch wird Cyclosporin A definiert als Cyclo-[[(E)-(2S,3R,4R)-3-hydroxy-4-methyl-2-(methylamino)-6-octenoyl]-L-2-aminobutyryl-N-methylglycyl-N-methyl-L-leucyl-L-valyl-N-methyl-L-leucyl-L-alanyl-D-alanyl-N-methyl-L-leucyl-N-methyl-L-leucyl-N-methyl-L-valyl]. Durch seine Verfügbarkeit begann eine neue Ära in der Transplantationsmedizin, denn mit seiner Hilfe konnte der Anteil an transplantierten Organen, die langfristig funktionsfähig bleiben, ganz erheblich gesteigert werden.

Schon mit dem ersten Cyclosporin-Präparat (Sandimmun, Fa. Sandoz) konnte bei Nierentransplantationen die Erfolgsrate etwa verdoppelt werden. Eine neue orale Zubereitung von Cyclosporin (Neoral, Fa. Sandoz später Novartis) mit höherer und zuverlässigerer Bioverfügbarkeit erlaubte ab den 90er Jahren eine bessere Dosierung und eine weitere Steigerung der Erfolgsrate. Trotz einiger neuer Wirkstoffentwicklungen ist Cyclosporin immer noch ein häufig eingesetztes Mittel in der Transplantationsmedizin

Auch Lungentransplantationen können heute prinzipiell erfolgreich durchgeführt werden, wenn die Patienten mit Cyclosporin A behandelt werden. Seit der Einführung des Wirkstoffs in die Therapie ist die Zahl der weltweit durchgeführten Lungentransplantationen sprunghaft gestiegen. Dies betrifft sowohl Einzellungentransplantationen - also die Transplantation eines Lungenflügels - als auch Doppellungentransplantationen. Für eine Lungentransplantation kommen normalerweise Patienten mit einer Lungenerkrankung im Endstadium in Betracht, bei denen die medikamentöse Therapie ausgeschöpft und deren Lebenserwartung aufgrund der Erkrankung nur noch gering ist. Indikationen zur Transplantation einzelner Lungenflügel sind z. B. bestimmte Formen von Emphysem und Fibrose, etwa die idiopathische pulmonale Fibrose. Beide Lungenflügel werden transplantiert bei Mukoviszidose, primärem Lungenhochdruck, Emphysem mit Globalinsuffizienz, häufigen schweren Infektionen, sowie idiopathischer pulmonaler Fibrose mit Komplikation durch wiederholte Infektionen.

Im Falle einer erfolgreichen Lungentransplantation kann die Lebensqualität der Patienten fast wieder auf ein normales Niveau steigen. Im Gegensatz zu Herz-, Nieren- oder Lebertransplantationen sind die Überlebenszeiten nach Lungentransplantation jedoch noch relativ gering und betragen im Durchschnitt nur etwa 5 Jahre. Dies könnte unter anderem damit zu tun haben, dass der Wirkstoff Cyclosporin nicht bei allen Patienten wirksam dosiert werden kann, sondern aufgrund von systemischen Nebenwirkungen wie Nierenfunktionsstörungen, erhöhte Serumspiegel von Kreatinin u. Harnstoff, Nierenschädigung mit Strukturveränderungen, z. B. interstitielle Fibrose, Anstieg von Bilirubin und Leberenzymen im Serum, Hypertrichose, Tremor, Müdigkeit, Kopfschmerzen, Gingivitis hypertrophicans, gastrointestinale Beschwerden wie Appetitlosigkeit, Bauchschmerzen, Übelkeit, Erbrechen, Durchfall, Gastritis, Gastroenteritis, Parästhesien, Brennen in Händen und Füßen, arterielle Hypertonie, Erhöhung der Blutfettwerte, Akne, Hautausschlag, allergische Hauterscheinungen, Hyperglykämien, Anämie, Hyperurikämie, Gicht, Gewichtszunahme, Ödeme, Magenulzera, Konvulsionen, Menstruationsstörungen, Hyperkaliämie, Hypomagnesiämie, Hitzewallungen, Hautrötung, Juckreiz, Muskelkrämpfe, Muskelschmerzen, Myopathie u. v. a. m.

Es wäre deshalb wünschenswert, Cyclosporin A beispielsweise nach einer Lungentransplantation, aber auch beim Vorliegen bestimmter anderer Indikationen, gezielt und gewebespezifisch zu verabreichen und dabei nur eine geringe systemische Bioverfügbarkeit des Wirkstoffs zu bewirken, um die Belastung der gesunden Gewebe mit dem Wirkstoff möglichst gering zu halten.

Eine geeignete Darreichungsform könnte auch zur Behandlung und Prävention von Erkrankungen, wie z.B. Asthma, idiopathische pulmonale Fibrose, Sarcoidose, Alveolitis und parenchymalen Lungenerkrankungen eingesetzt werden (siehe dazu: Drugs for the treatment of respiratory diseases, edited by Domenico Spina, Clive p. Page et. al., Cambridge University Press, 2003, ISBN 0521773210). Neue therapeutische Aspekte ergeben sich auch in der topischen Behandlung von möglichen autoimmun induzierten Erkrankungen, wie Neurodermitis, Psoriasis, unspezifischen Ekzemen, Hautwucherungen oder -Veränderungen, und zur Behandlung nach Hauttransplantationen.rEin interessantes Einsatzgebiet ist auch die Ophthalmologie, z.B. zur Behandlung nach Hornhauttransplantationen, der Keratokonjunktivitis oder anderer entzündlicher Augenerkrankungen, die auf eine antientzündliche Therapie mit z.B. Steroiden zum Teil nur unzureichend ansprechen. Es eignet sich auch zur Behandlung von Keratiden bei Tieren, wie z.B. Hunden.

In der Tat hat es bereits Bestrebungen gegeben, Cyclosporin lokal zu verabreichen, z. B. in Form öliger Augentropfen 1% und 2% (Rezeptur gemäß deutschem Arzneimittel Codex unter Verwendung von raffiniertem Erdnussöl als Lösungsvermittler) oder eines Aerosols. Allerdings scheitert dieser Ansatz bisher regelmäßig vor allem an der sehr geringen Wasserlöslichkeit des Wirkstoffs, die eine effiziente Verabreichung erheblich erschwert. So kann im Fall einer pulmonalen Applikation aus Verträglichkeitsgründen nicht wie bei der oralen Verabreichung auf bestimmte Hilfsstoffe zur Solubilisierung zurückgegriffen werden. Z. B. enthalten die Cyclosporin A enthaltenden Sandimmun Optoral Kapseln (Fa. Novartis) ein Mikroemulsionskonzentrat mit Ethanol, Propylenglykol und größeren Mengen an Tensiden und stellen somit eine Formulierung dar, die, würde man sie inhalieren, gravierende toxische Effekte hervorriefe.

Auch die zur Infusion verfügbare Zubereitung Sandimmun® Infusionslösungskonzentrat (Fa. Novartis) ist nicht inhalierbar: Als Hilfsstoffe enthält sie ausschließlich Ethanol und Poly(oxyethylen)-40-Rizinusöl. Sie kann auch nur deshalb infundiert werden, weil sie zuvor mit einer 0,9 %igen Kochsalzlösung oder einer 5 %igen Glukoselösung zu verdünnen ist, und zwar im Verhältnis 1 : 20 bis 1 : 100. Dadurch entstehen wiederum große Applikationsvolumina, die zwar infundiert, jedoch nicht inhaliert werden können.

In WO 00/45834 wird die Inhalation von aerosolisiertem Cyclosporin zur Verhinderung oder Bekämpfung von Abstoßungsreaktionen nach Lungentransplantation vorgeschlagen. Es wird empfohlen, eine Wirkstoffdosis von 15 bis 30 mg Cyclosporin A in die Lunge einzubringen. Als Wirkstoffträger soll Propylenglykol verwendet werden, was in dieser hochkonzentrierten Form zu erheblichen Irritationen führt, weshalb vor der Applikation, die Patienten eine Lidocianlösung zur Lokalanästhesie inhalieren. Aus neueren Untersuchungen(Akkar et. al, Posterpräsentation bei der NACF 2005) ist auch bekannt, daß Propylenglycol konzentrationsabhängig Calu-3 Zellen abtötet, die ein etabliertes Modell für Lungenepithelzellen darstellen (Steimer et al. Jour. Aerosol Med. 18 (2) pp.137-182, 2205). Aus physiologischen Gründen wäre deshalb eine überwiegend wässrige Zubereitung wünschenswert.

In EP 0 294 239 A1 wird eine wässrige Zubereitung von Cyclosporin für die pulmonale Applikation beschrieben. Zur Erhöhung der Löslichkeit enthält die Zubereitung ein α-Cyclodextrin. Allerdings ist der Solubilisierungseffekt für eine effiziente Inhalationstherapie bei weitem nicht ausreichend: es werden lediglich Cyclosporingehalte zwischen 0,1 und 2,0 mg/mL erzielt, insbesondere zwischen 0,2 und 1,5 mg/mL. Dies würde bedeuten, dass es mit einem konventionellen Vernebler ggf. Stunden dauern könnte, um auch nur eine einzige Dosis von 20 mg in die Lunge einzubringen.

In EP 0 504 760 A1 wird eine spezielle orthothrombische Kristallform von Cyclosporin A vorgestellt, die sich besonders für die Inhalation eignen soll. Allerdings wäre diese nur für Pulverinhalate oder für Zubereitung mit suspendiertem Wirkstoff relevant und nicht für wässrige Lösungen zur Verneblung. Pulverinhalatoren wiederum erfordern ein recht hohes Atemzugvolumen und sind wenig geeignet, um lungenkranke Patienten effizient zu behandeln. Darüber hinaus ist bekannt, daß Pulvermengen > 20 mg häufig zu Hustenreiz führen und der respirable Anteil bei den meisten Pulvermischungen mit Zunahme der Carrierkonzenträtion, wie z.B. Lactose, Trehalose, etc. abnimmt. Außerdem ist aufgrund aller vorliegenden in-vitro Daten fraglich, ob der sehr schwer lösliche Wirkstoff, wenn er in Form von suspendierten Teilchen in die Lunge gebracht wird, sich ausreichend in der geringen dort vorhandenen Menge an Schleim auflösen würde, was aber wiederum eine Voraussetzung seiner Wirksamkeit wäre. Prinzipiell Ähnliches gilt für die WO 99/42124, in der ein amorphes, flüssigkristallines Cyclosporin beschrieben wird.

WO 95/24892 beschreibt eine treibgashaltige Cyclosporinzubereitung, die in Form eines Dosieraerosols angewendet werden soll. Dosieraerosole stehen allerdings seit Jahren in der Kritik, da sie an der globalen Erwärmung beteiligt sind, und es ist fraglich, ob treibgashaltige Aerosole mittelfristig noch zulassungsfähig sein werden. Ähnliches gilt für WO 98/01147. Bekannt ist auch, daß der respirable Anteil abnimmt, wenn Wirkstoffe in Konzentrationen > 1 mg/ Hub appliziert werden und die Dosiergenauigkeit in-vivo sehr großen Schwankungen unterworfen ist. Bezugnehmend auf eine Lungendeposition von nur ca. 10% für ein Dosieraerosol kann gefolgert werden, daß mehr als 50 Hübe notwendig wären, um therapeutische relevante Cyclosporin Konzentrationen in die peripheren Bereiche der Lunge zu deponieren.

In WO 98/00111 wird eine liposomale Dispersion von Cyclosporin A zur Inhalation mit einen sehr hohen Phospholipidgehalt von bis zu 225 mg/mL vorgeschlagen. jedoch eine so hohe dynamische Viskosität hat, weshalb sie nicht effizient vernebelt werden kann. Ebenfalls eine liposomale Zubereitung von Cyclosporin A ist aus US 2003/0215494 bekannt Hier besteht die Erfindung allerdings darin, dass mit einer solchen Zubereitung Lungenmetastasen inhibiert werden sollen. Zu den technischen Problemen der Bereitstellung einer für die Inhalation besser geeigneten Zubereitung des Wirkstoffs leistet das Dokuments keinen Beitrag. In dem US-Patent 5,958,378 werden liposomale Cyclosporin Zubereitungen für die Vernebelung beschrieben, deren Viskosität allerdings so hoch ist, daß diese nicht mit einem elektronischen Schwingmembranvernebler, wie dem eFlow vernebelbar sind. Darüber hinaus wird für die Herstellung das organische Lösungsmittel Butanol verwendet, das trotz einem nachgeschalteten Lyophilisationsprozess nicht vollständig entfernt werden kann und Liposomen > 1 µm ergibt, die nicht mittels Sterilfiltration sterilisiert werden können und nur eine geringe Permeationsfähigkeit durch epitheliale Zellmembranen aufweisen.

Herkömmliche nicht-liposomale topische Zubereitungen, wie z.B. Cremes, Salben oder Lotionen zeigen bei Hauterkrankungen wie Neurodermitis oder Psorias eine unzureichende topische Wirksamkeit, weil durch Schuppung und Verhornung der Epidermis der Eindring- bzw. Penetrationseffekt unzureichend ist. Ebenso ist bekannt, dass in manchen Fällen bei diesen Erkrankungen auch liposomale Zubereitungen nicht automatisch eine verbesserte Hautpermeation zeigen, sondern in Abhängigkeit von der speziellen Zusammensetzung sowie der Größe und Beschaffenheit der Liposomen nur unwesentliche Verbesserungen brihgen.

### BESCHREIBUNG DER ERFINDUNG

Aufgabe der Erfindung ist die Schaffung einer Cyclosporin enthaltenden Zusammensetzung, welche die Nachteile des Standes der Technik nicht aufweist.

Die Aufgabe wird gelöst durch die Bereitstellung der Zusammensetzung gemäß. Anspruch 1. Weitere Lösungen und Ausführungsformen ergeben sich aus den übrigen Patentansprüchen.

Bei der efindungsgemäßen Zusammensetzung handelt es sich um eine flüssige wässrige Zubereitung, die eine therapeutisch wirksame Dosis eines Cyclosporins, eine erste löslichkeitsverbessernde Substanz ausgewählt aus der Gruppe der Phospholipide, und eine zweite löslichkeitsverbessernde Substanz, ausgewählt aus der Gruppe der nichtionischen Tenside enthält, wobei das Phospholipid eine Mischung natürlicher Phospholipide ist und wobei die Zusammensetzung das Cyclosporin in Liposomal Solubilisierter Form enthält. Ein besonders bevorzugtes Cyclosporin ist Cyclosporin A.

Vorzugsweise handelt es sich um eine Zusammensetzung, die den Wirkstoff Cyclosporin in liposomal solubilisierter Form enthält. Die Liposomen, die vor allem durch das enthaltene Phospholipid gebildet werden, sind vorzugsweise unilamellare Liposomen. Die Liposomen besitzen bevorzugt einen mittleren Durchmesser von maximal etwa 100 nm, gemessen als z-Average in einem Malvem ZetaSizer, und einem Polydispersitätsindex von maximal etwa 0.5, vorzugsweise von maximal etwa 0.4.

Die Herstellung der Liposomen erfolgt vorzugsweise ohne Zuhilfenahme von organischen Lösungsmitteln mit Wasser als Trägerflüssigkeit. Die Zubereitung ist vorzugsweise weitgehend isoton, und hat keinen negativen Einfluss auf den transepithelialen elektrischen Widerstand (TEER) in einem Calu-3 Lungenepithel Zellmodell, der ein Maß für die Verträglichkeit von Arzneistoff und Formulierung in Bezug auf die Beeinflussung der zellulären Vitalität darstellt und führt auch in humanen Lungenzellen zu keiner signfikanten Erhöhung von Interleukin-8, einem Entzündungsbiomarker.

Im Sinne dieser Erfindung ist eine pharmazeutische Zusammensetzung eine Zubereitung aus mindestens einem Wirkstoff und mindestens einem Hilfsstoff, bei dem es sich im einfachsten Fall z. B. um einen Träger wie Wasser handeln kann. Ein Wirkstoff ist eine Substanz oder ein Substanzgemisch, die bzw. das geeignet ist, die Gesundheit oder das Wohlbefinden eines Tieres oder eines Menschen direkt oder indirekt zu fördern oder zu unterstützen. Ein Wirkstoff kann eine diagnostische, prophylaktische oder therapeutische Funktion erfüllen, meist im oder am Körper des Tieres oder des Menschen, manchmal jedoch auch in-vitro, z. B. im Kontakt mit entnommenen Körperbestandteilen wie Zellen oder Körperflüssigkeiten.

Im vorliegenden Falle handelt es sich bevorzugt um eine kolloidale wässrige Lösung ohne organische Lösungsmittel, bestehend aus unilamellaren Liposomen mit einem Durchmesser von maximal 100 nm, in denen der Arzneistoff zumindest überwiegend gelöst vorliegt. Vorzugsweise ist Wasser das einzige in der Zubereitung enthaltene flüssige Lösemittel. Weiterhin ist bevorzugt, dass die Zubereitung in Form einer wässrigen Lösung oder einer wässrigen kolloidalen Lösung vorliegt, also als einphasiges flüssiges System. Ein solches System ist weitgehend frei von dispergierten Teilchen, die eine größere als eine kolloidale Teilchengröße besitzen. Konventionsgemäß werden Teilchen unterhalb etwa 1 µm als kolloidale Teilchen angesehen, die keine eigenständige Phase darstellen und keine physikalische Phasengrenze mit sich bringen. Manchmal werden auch Teilchen im Größenbereich knapp oberhalb 1 µm noch als kolloidal angesehen. Vorzugsweise jedoch ist die Erfindung weitgehend frei von Partikeln, die eindeutig nicht mehr dem kolloidalen Spektrum zuzuordnen sind, also z. B. von Teilchen mit einem Durchmesser von 1 µm oder mehr.

Die Zusammensetzung enthält eine therapeutisch wirksame Dosis eines Cyclosporins, bei dem es sich vorzugsweise um Cyclosporin A handelt. Cyclosporin A (auch Ciclosporin A) ist chemisch definiert als Cyclo-[[(E)-(2S,3R,4R)-3-hydroxy-4-methyl-2-(methylamino)-6-octenoyl]-L-2-aminobutyryl-N-methylglycyl-N-methyl-L-leucyl-L-valyl-N-methyl-L-leucyl-L-alanyl-D-alanyl-N-methyl-L-leucyl-N-methyl-L-leucyl-N-methyl-L-valyl], und ist ein zyklisches Peptid mit immunsuppressiver Wirkung. In diesem Zusammenhang schließt der Begriff "therapeutisch wirksam" auch eine prophylaktische Wirksamkeit mit ein. Die therapeutische Dosis ist in Abhängigkeit vom jeweiligen Anwendungsfall zu definieren. Je nach Art und Schwere der Erkrankung, Applikationsweg sowie Körpergröße und Zustand des Patienten ist eine therapeutische Dosis in einer dem Fachmann bekannten Weise festzulegen. So sind einige gängige Dosierungshinweise z. B. in der Fachinformation zu den Handelspräparaten der Marke Sandimmun^{®} der Novartis Pharma AG enthalten, bei denen es sich ebenfalls um Zubereitungen mit Cyclosporin A handelt. Die Erfindung soll allerdings auch dazu dienen, Cyclosporin auf anderen als den bisherigen Applikationswegen zu verabreichen, insbesondere per Inhalation nach Vemeblung der Zubereitung mit einem geeigneten Vernebler, und es wird notwendig sein, die Dosis des Wirkstoffs im Sinne dieser Anwendung nach gängigen Methoden zu adaptieren. Des weitem kann die erfindungsgemäße Zubereitung in derselben oder geringen Konzentrationen auch topisch auf die haut aufgetragen oder gesprüht werden oder in Auge und Ohr eingeträufelt werden.

Es wurde nun überraschend gefunden, dass Cyclosporin in einer wässrigen, flüssigen Zubereitung durch einen Gehalt an einem Phospholipid, wobei das Phospholipid eine Mischung natürlicher Phospholipide ist, und einem nichtionischen Tensid wirksam solubilisiert und gleichzeitig geschmacklich maskiert werden kann, fallweise sogar eine Verbesserung seiner Stabilität erfährt. Erfindungsgemäß enthält die Zusammensetzung also neben Cyclosporin und Wasser ein Gemisch von natürlichen Phospholipiden, wie z.B. Lipoid S 100 oder Phospholipon G 90, und ein nichtionisches Tensid, bei dem es sich vorzugsweise um ein Polysorbat handelt, insbesondere um Polysorbat 80.

Diese zweite oberflächenaktive Substanz wirkt mit dem Phospholipid synergistisch und erhöht nochmals die echte oder kolloidale Wasserlöslichkeit des enthaltenen Cyclosporins in statistisch signifikantem Ausmaß. Ein Tensid ist eine amphiphile bzw. oberflächenaktive Substanz oder ein Substanzgemisch mit oberflächenaktiven Eigenschaften. Tenside besitzen mindestens je eine eher hydrophile und eine eher lipophile Molekülregion. Es gibt monomere, niedermolekulare Tenside und solche, die eine oligomere oder polymere Struktur haben. Des Weiteren werden ionische und nichtionische Tenside unterschieden. Beispiele für geeignete Tenside im Sinne der Erfindung sind Polyoxyethylenalkylether, Polyoxyethylensorbitanfettsäureester wie z. B. Polyoxyethylensorbitanoleat, Sorbitanfettsäureester, Poloxamere, Vitamin E-TPGS (D-α-Tocopheryl-polyethylenglykol-1000-succinat), und Tyloxapol.

Als Phospholipid werden gegenwärtig Gemische von natürlichen Phospholipiden verwendet, zum Beispiel Lecithine wie die Handelsprodukte Phospholipon G 90, -100, oder Lipoid 90, S 100. Unter den nichtionischen Tensiden sind Polysorbate und Vit. E-TPGS bevorzugt, besonders Polysorbat 80.

Phospholipide sind amphiphile Lipide, die Phosphor enthalten. Auch unter der Bezeichnung Phosphatide bekannt, besitzen sie in der Natur insbesondere als doppelschichtbildende Bestandteile biologischer Membranen eine wichtige Rolle. Weit verbreitet und auch zu pharmazeutischen Zwecken häufig eingesetzt sind solche Phospholipide, die sich chemisch von der Phosphatidsäure ableiten. Bei dieser handelt es sich um ein (meist zweifach) acyliertes Glycerol-3-phosphat, bei dem die Fettsäurereste unterschiedlicher Länge sein können. Die Abkömmlinge der Phosphatidsäure sind z. B. die Phosphocholine oder Phosphatidylcholine, bei denen die Phosphatgruppe zusätzlich mit Cholin verestert ist, ferner Phosphatidylethanolamine, Phosphatidylinosite usw. Lecithine sind natürliche Mischungen verschiedener Phospholipide, die in der Regel einen hohen Anteil von Phosphatidylcholinen aufweisen. Bevorzugte Phospholipide sind erfindungsgemäß Lecithine sowie reine bzw. angereicherte Phosphatidylcholine wie Dimyristoylphosphatidylcholin, Dipalmitoylphosphatidylcholin und Distearoylphosphatidylcholin.

In einer weiteren bevorzugten Ausführung der Erfindung können toxikologsich unbedenkliche Stabilisatoren und Antioxidantien, wie Natrium-Ethylen-Diamin-Tetra-Essigäure (Na-EDTA, Tocopherole), Isotonisierungszusätze wie Natriumchlorid, Mannit, Trehalose oder Puffersalze (Citrat-, Carbonat, Phosphat-, Boratpuffer, etc), Geschmackskorrigentien wie Saccharin, Aspartam oder Minzöl zugegeben werden.

Die quantitative Zusammensetzung wird sich im Regelfall nach der Indikation richten. Im Allgemeinen ist der Cyclosporingehalt zwischen etwa 0.2 und 20 mg/ml, bevorzugt zwischen etwa 0.5 und 10 mg/ml, mehr bevorzugt zwischen etwa 1 und 5 mg/ml, am meisten bevorzugt zwischen etwa 1 und 4.5 mg/ml, zu wählen. Zur Behandlung von Asthma werden geringere Dosen diskutiert, die in einem Bereich von 0.25 - 5 mg/ml liegen können. Um die Vembelungszeit mit einem Kompressor-, Düsen- oder elektronischen Vernebler, wie z.B. dem AerX, AeroNeb Go, Omron U22, oder eFlow möglichst kurz zu halten, ist es vorteilhaft, kleinvolumige Lösungen (< 2 ml) in höherer Konzentration zu verwenden. Auch für die besonders bevorzugte Verwendung der Zusammensetzung, nämlich als Inhalationslösung zur Prophylaxe oder Therapie von Lungentransplantat-Abstoßungsreaktionen, ist es wünschenswert und im Sinne einer ausreichenden Patienten-Compliance erforderlich, dass der Gehalt an Cyclosporin möglichst hoch und dadurch die Inhalationszeit möglichst kurz ist. Vorzugsweise sollte der Gehalt an Cyclosporin - dies gilt insbesondere für Cyclosporin A - bei mindestens etwa 0.5 mg/mL liegen, z. B. zwischen etwa 0.5 und 10 mg/ml. Besser noch ist ein Gehalt von etwa 1 - 5 mg/mL, was durch die Verwendung der Merkmale der Erfindung auch erzielbar ist. In einer weiterhin bevorzugten Ausführung hat die Zusammensetzungen einen Gehalt an Cyclosporin A für die topische Anwendung auf der Haut oder zum Einträufeln in Auge und Ohr von 0.1 - 2% und zur Prophylaxe oder Behandlung respiratorischer Erkrankungen von 0.5 - 5 mg/mL oder auch mehr, z. B. einen Gehalt zwischen etwa 5-10 mg/mL,

Der erforderliche Gehalt der grenzflächenaktiven Hilfsstoffe richtet sich nach dem Gehalt an Cyclosporin A. Werden geringere Konzentrationen von 0.5 -1% Wirkstoff solubilisiert, so kann der Lecithin/Tensid Anteil proportional reduziert werden. Im Allgemeinen sollte der Phospholipidgehalt der Zusammensetzung zwischen etwa 0.2 und etwa 15 Gew.-% liegen, und vorzugsweise im Bereich von etwa 1 bis etwa 8 Gew.-%.

Das nichtionische Tensid sollte mit einem Gehalt von etwa 0.01 bis etwa 5 Gew.-% enthalten sein, und vorzugsweise sollte der Gehalt im Bereich von 0.1 bis 2 Gew.-% liegen, insbesondere im Fall, dass ein Polysorbat ausgewählt wird, z. B. Polysorbat 80.

Das Gewichtsverhältnis zwischen dem Phospholipid bzw. der Phospholipidkomponente und dem nichtionischen Tensid ist besonders wichtig für die solubilisierbare Menge an Cyclosporin pro Volumeneinheit. Bevorzugt wird ein Verhältnis zwischen etwa 15: 1 und 9 : 1, insbesondere zwischen etwa 14 : 1 und 12 : 1, also z. B. im Bereich von etwa 13 : 1. Diese Vorzugsbereiche gelten ebenfalls ganz besonders für den Fall, dass ein Polysorbat wie z. B. Polysorbat 80 als nichtionisches Tensid ausgewählt ist.

Das Gewichtsverhältnis zwischen den beiden solubilisierenden Hilfsstoffkompnenten, also dem Phospholipid und dem nichtionischen Tensid einerseits, und dem Cyclosporin andererseits, ist im Allgemeinen zwischen etwa 5 : 1 und etwa 20 : 1 zu wählen. In gegenwärtig bevorzugten Ausführungen liegt das Verhältnis bei etwa 8 : 1 bis etwa 12 : 1, z. B. bei etwa 10 : 1. Besonders bevorzugt ist eine Zusammensetzung, von Cyclosporin A zu dem Lipoid S100 / Tensid Gemisch von 1: 9. mit einem Gehalt von bis zu 0.5 Gew.-% solubilisiertem Cyclosporin A, so daß sich ein Gemischverhätnis wie folgt ergibt: Cyclosporin : Lipoid S 100: Polysorbat 80 = 1: 9: 0.69 und man unilamillarrre Liposomen mit einem Cyclosporin Gehalt von insgesamt etwa 4 bis 6 Gew.-%, z. B. etwa 5 Gew.-% erthält.

Die untenstehende Tabelle zeigt einige Beispiele für Mengenverhältnisse, bei denen Cyclosporin A liposomal optimal solubilisiert werden kann:

| Cyclosporin A | Lipoid S 100 | Polysorbat 80 | Aqua purificata | NaCl |
|---|---|---|---|---|
| 0.1% | 0.9% | 0.07% | 98.77% | 8% |
| 1% | 9.0% | 0.7% | 87.7% | 8% |
| 5% | 45% | 3.5% | 38.5% | 8.0% |

Die erfindungsgemäße Zusammensetzung hat den Vorteil, dass sie einen relativ hohen Gehalt an einem schwerlöslichen Cyclosporin in solubilisierter Form enthalten kann. Gleichzeitig liegt das Cyclosporin geschmacksmaskiert vor, was für alle oralen, oromukosalen, nasalen sowie pulmonalen Anwendungsformen besonders vorteilhaft ist, so auch für die besonders bevorzugte Verwendung der Zubereitung zur Herstellung eines Arzneimittels zur topischen Therapie der Haut, an Auge, Nase und Ohr und besonders zur inhalativen Prophylaxe oder Behandlung von Lungentransplantat-Abstoßung.

Die Zusammensetzung kann weitere, für die jeweilige Anwendung sinnvolle und übliche pharmazeutische Hilfsstoffe enthalten. Geeignete Hilfsstoffe sind dem Fachmann bekannt. Optional können beispielsweise pH-Korrigenzien zum Einstellen des pH-Werts enthalten sein, wie etwa physiologisch akzeptable Basen, Säuren, oder Salze, ggf. auch als Puffermischung. Physiologisch akzeptabel bedeutet in diesem Zusammenhang nicht, dass einer der Hilfsstoffe allein und unverdünnt verträglich sein muss, was z. B. bei Natronlauge nicht der Fall wäre, sondern in seiner in der Zubereitung enthaltenen Konzentration.

Geeignete pH-Korrigenzien zum Einstellen des pH-Werts sind auch in Berücksichtigung des beabsichtigten Applikationsweges auszuwählen. Beispiele für ggf. geeignete Hilfsstoffe dieser Gruppe sind Natronlauge, basische Salze von Natrium, Calcium, oder Magnesium wie z. B. Citrate, Phosphate, Acetate, Tartrate, Laktate usw., Aminosäuren, saure Salze wie Hydrogenphosphate oder Dihydrogenphosphate insbesondere des Natriums, ferner organische und anorganische Säuren wie z. B. Salzsäure, Schwefelsäure, Phosphorsäure, Citronensäure, Cromoglycinsäure, Essigsäure, Milchsäure, Weinsäure, Bernsteinsäure, Fumarsäure, Lysin, Methionin, saure Hydrogenphosphate mit Natrium oder Kalium usw.

In einer der vorteilhaften Ausführungen der Erfindung ist die Zusammensetzung - mit oder ohne pH-Korrigenz - auf einen neutralen oder sauren pH-Wert eingestellt. Vorzugsweise liegt der pH-Wert im Bereich von maximal etwa 8,5, oder im Bereich von etwa 2,5 bis etwa 7,5. Wenn z. B. Für eine pulmonale oder parenterale Applikation ist ein pH-Wert von etwa 4 bis etwa 7,5 zu bevorzugen, sofern sich dieser mit anderen Notwendigkeiten der jeweiligen Formulierung, wie z.B Stabilitätsaspekte vereinbaren lässt. Besonders bevorzugt ist eine Zusammensetzung, die mit einem Phosphatpuffer im Bereich von pH 6.7 -7.5 und insbesondere im Bereich von pH 6.7 bis 7.3 gepuffert ist, wodurch die Stablität der liposomalen Formulierung deutlich verbessert und das Auftreten von unerwünschtem Lysolecithin im Laufe der Lagerung wirksam reduziert werden kann (siehe Beispiel 4).

Des Weiteren kann die Zubereitung osmotisch aktive Hilfsstoffe zur Einstellung einer gewünschten Osmolalität enthalten, wie sie für bestimmte Anwendungen wie für die parenterale Injektion oder für die Inhalation oder sonstige topische Applikation im Sinne einer guten Verträglichkeit wichtig ist. Derartige Hilfsstoffe werden häufig als Isotonisierungsmittel bezeichnet, auch wenn ihr Zusatz nicht unbedingt zu Isotonie führen muss, sondern auch nur zu einer Annäherung an die physiologische Osmolalität mit der Zielsetzung einer best möglichen physiologischen Verträglichkeit.

Ein besonders häufig verwendetes Isotonisierungsmittel ist Natriumchlorid, doch ist es nicht in jedem Fall geeignet. In einer der vorteilhaften Ausführungen der Erfindung ist kein Kochsalz enthalten, wobei natürlich die ubiquitären Natriumchloridmengen, die auch in pharmazeutischen Wasserqualitäten enthalten sein können, ausgenommen sind. In einer anderen Ausführung ist als Isotonisierungsmittel ein weitgehend neutrales Salz in der Zubereitung enthalten, bei dem es sich nicht um Kochsalz handelt, sondern z. B. um ein Natriumsulfat oder Natriumphosphat. In diesem Falle sind allerdings andere Salze als Natriumsalze noch mehr zu bevorzugen. So ist von bestimmten Calcium- und Magnesiumsalzen bekannt, dass sie bei der Inhalation von Wirkstofflösungen einen positiven bzw. unterstützenden Einfluss haben können, möglicherweise weil sie selbst den lokalen Irritationen durch die Applikation entgegenwirken und einen bronchodilatatorischen Effekt ausüben, der zur Zeit in der klinischen Literatur postuliert wird (z. B. R. Hughes et al., Lancet. 2003; 361 (9375): 2114-7), und/oder weil sie die Haftung von Keimen an den Proteoglykanen der Schleimhäute des Respirationstrakts hemmen, so dass die mucociliäre Clearance als natürlicher Abwehrmechanismus des Organismus gegen die Erreger indirekt unterstützt wird (K. W. Tsang et al., Eur. Resp. 2003, 21, 932-938). Vorteilhaft ist z. B. Magnesiumsulfat, das eine ausgezeichnete pulmonale Verträglichkeit besitzt und bedenkenlos inhaliert werden kann, sowie Calciumchlorid (1-10 mMol)

Alternativ zu den neutralen Mineralsalzen können als Isotonisierungsmittel auch physiologisch unbedenkliche organische Hilfsstoffe eingesetzt werden. Besonders geeignet sind wasserlösliche Substanzen mit relativ geringer Molmasse, z. B. mit einer Molmasse von unter 300, oder besser noch unter 200, und mit einer entsprechend hohen osmotischen Aktivität. Beispiele für derartige Hilfsstoffe sind Zucker und Zuckeralkohole, insbesondere Mannitol und Sorbitol, Xylitol, Trehalose,

Die Einsatzmenge des gewählten Isotonisierungsmittels ist jeweils so abzustimmen, dass sich unter Berücksichtigung der übrigen in der Zusammensetzung enthaltenden Bestandteile eine Osmolalität von mindestens etwa 150 mOsmol/l ergibt. Weiterhin bevorzugt ist eine Osmolalität im Bereich von etwa 150 bis 800 mOsmol/l. In weiteren Ausführungen hat die Zubereitung eine Osmolalität von etwa 250 bis etwa 600 mOsmol/l, oder von etwa 250 bis 400 mOsmol/l.

Wenn allerdings ein möglichst hoher Gehalt an Cyclosporin angestrebt wird und demzufolge auch ein relativ hoher Gehalt an löslichkeitsverbessernden Hilfsstoffen eingesetzt werden muss, ist anzunehmen, dass auch ohne den Zusatz eines separaten Isotonisierungsmittel die Osmolalität der Zusammensetzung bereits im gewünschten Bereich oder sogar darüber liegen kann, so dass die Verwendung eines Isotonisierungsmittels nicht notwendig ist.

Da in der Zusammensetzung Tenside als löslichkeitsverbessernde Substanzen enthalten sind, hat dieses natürlich auch einen Einfluss auf die Oberflächenspannung der Zubereitung. Diese kann vor allem für eine pulmonale Anwendung von Bedeutung sein. In einer bevorzugten Ausführung besitzt die Zubereitung unter Normalbedingungen, d. h. bei Raumtemperatur und unter normalem Druck eine Oberflächenspannung von etwa 25 bis 75 mN/m, um eine effiziente Vemeblung mit einem hohen Anteil an lungengängigen Tröpfchen mit einem Durchmesser von höchstens 5 µm mit Hilfe gängiger Vernebler zu erlauben.

Soll die Zubereitung allerdings für den Einsatz in bestimmten Verneblertypen angepasst werden, kann die Oberflächenspannung auf bestimmte Werte, z. B. auf etwa 30 bis etwa 65 mN/m eingestellt werden. Noch mehr ist eine Oberflächenspannung von etwa 30 bis etwa 45 mN/m gegenwärtig bevorzugt, was aber nicht immer erreicht werden kann, wenn andere Formulierungsparameter im Vordergrund stehen. Dagegen sollte eine Oberflächenspannung von ca. 25 mN/m nicht unterschritten werden.

Die durch ein Tensid herabgesetzte Oberflächenspannung kann dazu beitragen, die Spreitung des Aerosols in der Lunge zu verbessern, was wiederum einen positiven Einfluss auf die Wirksamkeit der Anwendung haben kann.

Auch die Viskosität der Zusammensetzung kann im Falle einer Inhalativen Anwendung von Bedeutung sein. In einer der für eine Vemeblung bevorzugten Ausführungen besitzt die Zusammensetzung der Erfindung unter Normalbedingungen eine dynamische Viskosität von etwa 1,0 bis 3,0 mPa·s. In einer weiteren bevorzugten Ausführung liegt die Viskosität zwischen etwa 1,2 und 2 mPa·s.

Des Weiteren kann die Zubereitung weitere übliche Hilfsstoffe wir Stabilisatoren, Antioxidantien, Geschmacksverbesserer, Aromen, Süßungsmittel, Farbstoffe usw. enthalten, wie sie dem Fachmann zur Formulierung von Zubereitungen je nach Anwendungszweck zur Verfügung stehen.

Die liposomale erfindungsgemäße Zubereitung kann durch Hochdruckhomogenisation hergestellt werden. Beispielsweise können die wasserlöslichen Komponenten in Form einer wässrigen Lösung vorgelegt werden, in der das Cyclosporin dispergiert wird und danach in einem Hochdruckhomensiationsprozess in Liposomen überführt werden, wobei durch Einstellung von Druck und Zyklenzahl die Größe der Liposomen und der Polydispersitätsindex einstellbar sind.

Die Abfüllung der erfindungsgemäßen Zusammensetzung oder des Konzentrats erfolgt vorzugsweise aseptisch in Einzel- oder Mehrdosenbehältnisse. Geeignete Primärverpackungen sind z. B. Polypropylen- oder Polyethylen-Vials (PP-/PE-Vials) und Cycloolefin-Copolymer-Blister (COC-Blister) oder auch Polytherephtalat (PET-Blister) Alternativ dazu können auch Behältnisse aus anderen Polymeren oder Copolymeren Anwendung finden, die sich für einen Blow-Fill-Seal-Prozess oder thermoplastisches Verfahren eignen. Versiegelte Kunststoffbehältnisse wie PP- oder PE-Vials lassen sich beispielsweise vorteilhaft mit dem Blow-Fill-Seal-Verfahren in einem integrierten Prozess formen, befüllen und versiegeln. Die so hergestellten Behältnisse sind insbesondere für flüssige Füllgüter mit einem Volumen ab etwa 0,2 ml geeignet und eigenen sich in kleinen Volumina zum Einträufeln in Auge und Ohr. Für Anwendungen mit einem Vernebler oder zur topischen Anwendung auf der Haut, bei denen unterschiedliche Wirktoffmengen benötigt werden, können idealerweise Volumina von 0.2 - 5 ml Inhalt abgefüllt werden, vorzugsweise 0.5 bis 5 ml. Besonders patientenfreundlich können sie mit einem Verschluss geformt werden, der durch Drehen oder Abknicken entfembar ist und durch eine besondere Ausformung nicht scharfkantig ist und eine tropfenförmige, gut dosierbare Entnahme erlaubt.

Alternativ kann die Öffnung, durch die der flüssige Inhalt entnehmbar ist, auch so ausgestaltet sein, dass sie auf einen Luer-Anschluss oder Luer-Lock-Anschluss passt. So kann die Öffnung rund gestaltet sein und einen Durchmesser besitzen, der weitgehend dem Außendurchmesser eines männlichen Luer-Anschlusses entspricht. Auf diese Weise könnte eine gewöhnliche Spritze mit Luer-Anschluss mit dem Behältnis schlüssig verbunden werden, beispielsweise um den Inhalt des Behältnisses aufzunehmen und in einen Vernebler zu transferieren, oder um den Inhalt des Behältnisses mit dem Inhalt der Spritze zu mischen und anschließend in einen Vernebler zu geben. Als weitere Alternative kann vorgesehen sein, dass das Kunststoffbehältnis so beschaffen ist, dass es nach Entfernung des Verschlusselements mit einem für die Zufuhr von Flüssigkeit vorgesehenen Anschlussstück eines entsprechend adaptierten Verneblers weitgehend schlüssig verbindbar ist, wodurch eine direkte Einfüllung der Zubereitung in das Reservoir des Inhalators ermöglicht ist.

Kunststoffbehältnisse dieser Art sind weiterhin deshalb vorteilhaft, weil sie leicht mit Prägungen versehen werden können, die auch blinden Menschen die Identifikation eines Produktes erlaubten. Hierdurch kann einerseits auf Papieretiketten verzichtet werden, was wünschenswert ist, um die Migration von Bestandteilen des Klebers, des Papiers oder der Druckfarbe durch die Behältniswand in die Zubereitung zu vermeiden. Andererseits können wichtige Information durch eine solche Prägung auch sehbehinderten Patienten zugänglich gemacht werden. Die Prägung kann verschiedene Informationen enthalten, z. B. eine Chargenbezeichnung, ein Haltbarkeitsdatum, eine Produktbezeichnung, Hinweise für die Anwendung, oder auch eine oder mehrere Volumen- oder Dosismarken. Insbesondere für pädiatrische Patienten, bei denen oftmals eine flexible Dosierung nach Alter oder Körpergröße wünschenswert ist, kann eine Mehrzahl von Volumenmarken zur erleichterten Entnahme der gewünschten Dosis ohne weitere Hilfsmittel dienen, wodurch das Risiko von Dosierungsfehlern herabgesetzt werden kann.

In einer weiteren Variante der Erfindung sind Mehrdosenbehältnisse vorgesehen, die eine Zubereitung wie oben beschrieben enthalten und die des Weiteren so gestaltet sind, dass sie die aseptische Entnahme einer Einzeldosis gestatten. Das Mehrdosenbehältnis kann also wie ein Vial oder eine Infusionsflasche ein Glas- bzw. Kunststoffbehältnis mit einem mit einer Kanüle durchstechbaren Verschluss aus einem Elastomer sein, oder es kann sich um ein komplex aufgebautes Gefäß mit Dosier- und Entnahmevorrichtung handeln., wie dies von konservierungsmittelfreien Nasensprays her bekannt ist, so daß die efindungsgemässe Zubereitung nicht konserviert werden muß und in die Nase oder andere körperhöhlen eingesprüht oder auf die Haut aufgesprüht werden kann, wie das von der Applikation zur Behandlung von Fußpilz bekannt ist. Ein konservierungsmittelfreies Pumpspray bietet gerade bei Behandlung der Psoriasis oder Neurodermitis große Vorteile, weil man damit die Liposomen recht homogen und hygiensich einwandfrei auf die beschädigte und entzündete Haut aufbringen kann.

Einer der besonderen Vorteile von Mehrdosenbehältnissen im Zusammenhang mit Zubereitungen für die Inhalation ist die Flexibilität, die problemlos eine individuelle Dosisanpassung gestattet, ohne dass dadurch wesentliche Mengen an Zubereitung verworfen werden müssen, so wie dies bei Einzeldosisbehältnissen nach deren Öffnen der Fall ist. In Krankenhäusern und Pflegeeinrichtungen können auf diese Weise Patienten durch individuelle Dosisanpassung gleichzeitig besonders effizient und potentiell kostengünstig versorgt werden. Ebenso lassen sich spezielle Anforderungen an die Therapie einzelner Patienten besonders leicht berücksichtigen.

Wenn die Zusammensetzung im Endbehältnis nicht sterilisiert werden kann, ist sie vorzugsweise durch ein aseptisches Verfahren in die Behältnisse abzufüllen.

Für die bevorzugt vorgesehene Aerosolisierung der Zubereitung kann grundsätzlich jeder in der Therapie einsetzbare Vernebler verwendet werden. Die altbewährten Düsenvernebler sind prinzipiell ebenso geeignet wie neuere Ultraschall- oder piezoelektrische Vernebler. Der Vorteil der Düsenvernebler ist, dass sie bereits einen sehr großen Verbreitungsgrad besitzen und relativ kostengünstig zu beschaffen sind. Viele Patienten sind bereits im Umgang mit gängigen Düsenvernebler versiert. Einige Düsenvernebler der jüngeren Generation (z. B. PARI LC PLUS® und PARI LC SPRINT®) verwenden Mechanismen, mit denen die Verneblung an den Atmungsrhythmus des Patienten angepasst wird, so dass ein möglichst großer Anteil des erzeugten Aerosols auch für die Inhalation verfügbar ist.

Besonders bevorzugt jedoch ist die Aerosolisierung der Zubereitung mit Hilfe eines modernen piezoelektrischen oder elektronischen Schwingmembranverneblers, insbesondere mit einem Vernebler wie dem Typ eFlow^{™} der Firma PARI. Der besondere Vorteil für den Patienten bei der Verwendung dieses Geräts (oder eines ähnlichen Geräts) ist die gegenüber alternativen Methoden erheblich verkürzte Inhalationszeit. Das Gerät aerosolisiert nicht nur eine größere Menge an Flüssigkeit pro Zeiteinheit, es erzeugt auch ein qualitativ besonders hochwertiges Aerosol mit hohem Anteil an kleinen, lungengängigen Aerosoltröpfchen mit einem engen Tröpfchenverteilungsspektrum sprektrum, die typischweise eine geometrischen Standardabweichung < 1.6 aufweisen. Andere potentiell geeignete Schwingmembranvemebler sind z. B. die Geräte AeroNeb Pro oder -Go, ProDose, oder I-Neb.

Entscheidend für den therapeutischen Erfolg ist die zuverlässige und adäquate Verfügbarkeit des Wirkstoffs in der Lunge. Patientengerecht ist es, dies innerhalb eines akzeptablen Zeitraums zu bewirken. Von Patienten werden möglichst kurze Inhalationszeiten bevorzugt, und Inhalationszeiten von mehr als etwa 10 Minuten könnten die Compliance bereits negativ beeinflussen. Sinnvoll ist es auch, die Inhalation entweder durch kontinuierliche Inhalation oder mittels einem "breath trigger" oder einem "guided breathing pattem" vorzunehmen, wie dies z.B. mit einem Vernebler vom Typ eFlow in Verbindung mit einem Akita Inhalationsgerät (Fa. InaMed) möglich ist. Besonders bevorzugt ist ein Atemmanöver das sich durch eine lansame tiefe Inhalation über 4 - 10 sec erstreckt, einem Anhalten des Atems bis zu 10 Sekunden und einem raschen Ausatmen.

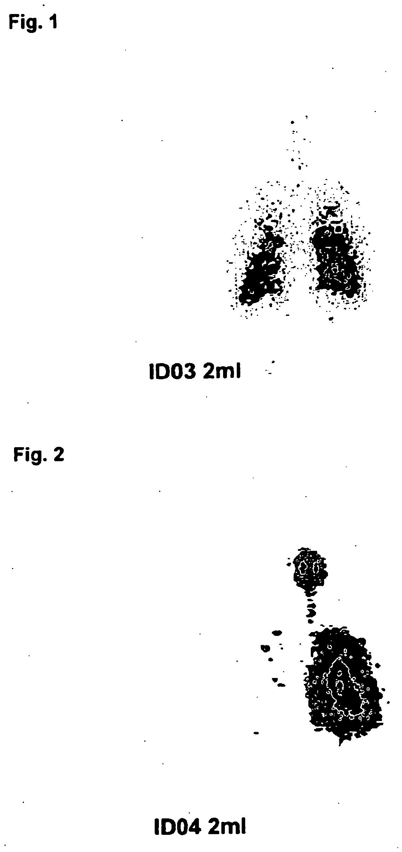

Wie aus Beispiel 4 und Figur 1 ersichtlich ist, wird der Wirkstoff sehr uniform in der Lunge verteilt, wobei jeweils ca. 50% zentral und peripher deponiert werden, was theapeutisch sinnvoll ist. Würde zuviel peripher deponiert werden, käme es zu einer verstärkten systemsichen Absorption, die bekannterweise in hohen Konzentrationen zu Nierenschäden führen kann. Idealerweise sollte das Verhältnis von zentraler zu peripherer Deposition abhängig vom Krankheitsbild z.B. zur Verhinderung einer bronchioltis obliterans und nachgeschalteten Abstoßung transplantierten lungen bei einem Verhältnis von zentral zu peripher = 30 : 70 bzw. 50 : 50 bzw. 70 : 30 liegen.

Doch auch die nasale, orale, ophthalmische, mukosale, parenterale oder topische Anwendung der Zusammensetzung der Erfindung kann im Einzelfall vorteilhaft sein. Die Applikation kann durch Auftragen, Einträufeln, Aufsprühen oder Einsprühen der Zusammensetzung erfolgen, die sich in ersten Humanversuchen als ausgesprochen gut verträglich herausgestellt hat.

Aufgrund seiner immunsuppressiven Wirkung ist Cyclosporin in ganz unterschiedlichen Indikation einsetzbar, z. B. für die Prophylaxe und Therapie von Transplantat-Abstoßung nach ganz unterschiedlichen Transplantationen (Niere, Leber, Herz, Herz-Lunge, Lunge, Pankreas, Knochenmark usw.), Prophylaxe und Therapie von Graft-versus-Host-Krankheit, Therapie der endogenen Uveitis, der manifesten, nichtinfektiösen Uveitis intermedia öder posterior, Behcet-Uveitis, der schweren und resistenten Psoriasis, insbesondere vom Plaque-Typ, beim nephrotischen Syndrom infolge glomerulärer Krankheiten wie glomerulärer Minimalveränderungen, fokaler segmentaler Glomerulosklerose oder membranöser Glomerulonephritis, der schweren aktiven rheumatoiden Arthritis, therapieresistenten Formen einer länger bestehenden atopischen Dermatitis sowie anderen Erkrankungen, die eine lokale oder systemische Immunsuppression angezeigt erscheinen lassen, wie z.B. bei der Behandlung von Asthma oder bei Augenerkrankungen. Neueren Erkenntnissen zufolge wirkst Cyclosporin auch als Pump-efflux inhibitor und verhindert dadurch, daß Krebszellen cytostatisch wirksame Medikamete herauspumpen.

Eine weitere Anwendung der erfindungsgemäßen Zusammensetzung ist die Prophylaxe oder Behandlung von Erkrankungen der Haut, insbesondere von Psoriasis, Neurodermitis, Ekzemen, oder Abstoßungsreaktionen nach einer Hauttransplantation. Die Behandlung kann z. B. dadurch geschehen, dass die Zusammensetzung in ein Pumpspray abgefüllt und als Spray auf die erkrankte Haut aufgetragen wird.

Wie weiter oben beschrieben, ist eine der besonders bevorzugten Anwendungen der erfindungsgemäßen Zusammensetzungen die Prophylaxe und Therapie der Lungentransplantat-Abstoßung sowie das Verhindern bzw. Verzögern des Auftretens einer Boncholitis obliterans. Diese Erkrankung tritt in vielen Fällen auch nach einer Knochenmarks- oder Stammzelltransplantation auf, weshalb eine Inhalation als besonders geeignet erachtet wird, Krankheitsbilder, wie z.B: Bronchiolitis obliterans zu verhindern bzw. zu behandeln. Für diese Anwendung ist die Zusammensetzung in Form eines Aerosols zu inhalieren. Die lokale Therapie hat gegenüber einer systemischen (z. B. über die orale Verabreichung) den Vorteil, dass der Wirkstoff gezielt zum Wirkort gebracht wird, was einerseits die Wirksamkeit erhöhen kann, andererseits die systemische Belastung des Organismus herabsetzt und die Verträglichkeit der Therapie verbessert. Durch die liposomale Verpackung des Wirkstoffs und die Größe der Liposomen von maximal 100 nm im Mittel kann der Wirkstoff aus den Luftwegen durch die epitheliale Lungenzellschicht besonders gut in das Lungengewebe permeieren und dort wirksam werden. In einer weiteren Variante werden Liposomen mit einem mittleren Durchmesser von 30 bis 80 nm bevorzugt.

Um eine lokale Inhalationstherapie der Lunge effizient zu gestalten, sollten die oben diskutierten Vorzugsmerkmale unter Berücksichtigung des Vemeblers, der zur Applikation bestimmt ist, so ausgewählt werden, dass die Erzeugung eines Aerosol mit einer möglichst großen Fraktion an lungengängigen Tröpfchen bevorzugt wird. Insbesondere der Anteil der Tröpfchen unterhalb einer Grenze von etwa 2-4 µm Durchmesser sollte so groß wie möglich sein. Besonders bevorzugt ist ein elektronischer Schwingmebranvernebler mit einer perforierten Edelstahlmembran, die ca. 4000 Poren einer definierten Größe enthält mit der ein Aerosol mit definiertem Partikelspektrum schnell und effizient erzeugt werden kann (Martin Knoch & Manfred Keller. The customized electronic nebuliser: a new category of liquid aerosol drug delivery systems. Expert Opin. Drug Deliv. (2005) 2 (2): 377-390). Die erfindungsgemäße liposomale Zubereitung kann damit ohne Zerstörung der Liposomen entweder in einem kontinuierlichen oder Atemzug-gesteuerten oder -geführten Aerosolerzeugungsmodus oder einer über eine Beatmungsmaschine so inhaliert werden, daß ein sehr hoher Wirkstoffanteil in kurzer Zeit gezielt in der Lunge deponiert werden kann. Der Vorteil dieses Inhalationssystems liegt vor allem darin, daß die Dosis, die vom Mundstück inhaliert werden kann größer 50% ist und bis zu 98% der erzeugten Tröpfchen einen Durchmesser kleiner 5 µm und bis zu 80% einen mittleren geometrischen Durchmesser kleiner 3.5 µm aufweisen, so daß der Wirkstoff gezielt in die distalen Bereiche der Lunge mit höherer Effizienz als bei Kompressor-Düsenvemeblen deponiert werden kann. Die Zusammensetzung kann einen oder mehrere weitere Wirkstoffe enthalten. Ein zusätzlicher Wirkstoff kann beispielsweise aus der Gruppe der Immunmodulatoren, Interferone, steroidale und nicht steroidale anti-inflammatorisiche Wirkstoffe, Heparinoide, Betaagonisten, Anticholinergika, Endothelin- und Phosphordiesterasehemmer, Antibiotika, Antimykotika, antiviralen Substanzen und Zytostatika ausgewählt sein. Alternativ kann eine Kombinationstherapie dadurch erreicht werden, dass die erfindungsgemäße Zusammensetzung als Wirkstoff nur Cyclosporin enthält, aber in Kombination mit einer anderen Zubereitung verabreicht wird, die einen anderen Wirkstoff enthält.

Die Verabreichung der erfindungsgemäßen Zusammensetzung, insbesondere die inhalative Verabreichung, kann ohne Prämedikation erfolgen. Insbesondere kann die Verabreichung ohne Prämedikation mit Lokalanästhetika wie z.B. Lidocain und/oder ohne Prämedikation mit Bronchiodilatoren wie z.B. Salbutamol erfolgen.

Vorzugsweise ist die Zusammensetzung steril, insbesondere wenn sie für die pulmonale, parenterale oder ophthalmische Applikation bestimmt ist. Ferner ist sie vorzugsweise weitgehend frei von festen Partikeln, die größer als etwa 3 µm sind. Es ist vorteilhaft, wenn z. B. der gesamte enthaltene Wirkstoff in liposomal solubilisierter Form vorliegt. Dementsprechend sollten feste Wirkstoffpartikel weitgehend abwesend sein, vor allem feste Wirkstoffpartikel mit einem Durchmesser von mehr als etwa 500 nm. Vorteilhaft sind auch Zusammensetzungen, die weitgehend frei von festen Partikeln irgendeiner Substanz mit einem Durchmesser von mehr als 500 nm sind.

Wie bereits erwähnt, kann die Zusammensetzung als Arzneimittel verwendet werden, z. B. zur Prophylaxe oder Behandlung von Autoimmunerkrankungen, Hautkrankheiten, nach Transplanationen oder Erkrankungen der Sinnesorgane (Augen, Nase, Ohr), Befindlichkeitsstörungen, und Lungenerkrankungen, z.B. Asthma, chronisch obstruktiver Bronchitis, parenchymalen, fibrotischen und interstitiellen Lungenerkrankungen oder -entzündungen, Lungenkrebs, und vorzugsweise zur Prävention und Behandlung akuter oder chronischer Transplantat-Abstoßungsreaktionen und deren Folgeerkrankungen wie Bronchiolitis obliterans, insbesondere nach Lungen-, Herz-, Knochenmarks-, oder Stammzelltransplantation, besonders bevorzugt nach Lungentransplantation. Sie kann ferner zur Wirksamkeitsverbesserung anderer Medikamente, insbesondere von Zytostatika verwendet werden, bei denen durch Cyclosporin durch den "Efflux pump inhibition"-Effekt ein additiver oder synergistischer Effekt erreichbar ist.

Die erfindungsgemäße pharmazeutische Zusammensetzung weist u.a. folgende Vorteile auf:
- Die Herstellung der Liposomen in einem Einstufenprozess kann auch im Großmassstab bis 1000 kg mittels Hochdruckhomogenisation erfolgen und eine Entkeimung durch eine nachgeschaltete Sterilfiltration mit einem Porendurchmesser von 0.22 µm ist möglich.
- Die Liposomen können über die Atemwege gut in das Lungenegewebe diffundieren.
- Es muß nur 1 - 3 mal täglich und besonders bevorzugt nur 1 - 2 täglich inhaliert werden, um einen therapeutischen Effekt zu erreichen.
- Die Zusammensetzung weist einen Retardeffekt am Zielorgan auf und muß in bestimmten Fällen nur 1 - 4 mal wöchentlich und besonders bevorzugt nur jeden zweiten Tag inhaliert werden.
- Die Zusammensetzung kann bei Kühlschranklagerung (4 - 8°C) mindestens 12 Monate und besonders bevorzugt bis zu 36 Monaten gelagert werden.
- Die Zusammensetzung kann auch in Beatmungsmaschinen oder in Verbinung mit Systemen für ein gesteuertes Atemmanöver, wie der eFlow-Akita eingesetzt werden.

Die Erfindung soll des Weiteren in einigen wichtigen Aspekten und Ausführungen durch die nachfolgenden Ausführungsbeispiele illustriert werden. Weitere Ausführungen sind dem Fachmann anhand der Beschreibung und der Patentansprüche zugänglich.

### Beispiel 1: Lösung zur topischen Behandlung zum Aufsprühen auf die Haut

| | **Konzentration [w/w%]** | **Funktion** |
|---|---|---|
| Cyclosporin A | 0.50 | Wirkstoff |
| Tween 80 | 0.35 | Löslichkeitsverbesserer |
| Phospholipon G 90 | 4.50 | Löslichkeitsverbesserer |
| NaCl | 0.5 | Isotonisierungsmittel |
| Dexpanthenol | 5.0 | Hautschutzstoff |
| Tocopherolacetat | 0.05 | Antioxydans |
| Natriumcitrat | 0.05 | Puffersubstanz |
| Citronensäure | 0.04 | Puffersubstanz |
| Wasser für Injektionszwecke | ad 100.0 | Lösungsmittel |

Die in der voranstehenden Tabelle angegebenen wasserlöslichen Hilfsstoffe (Natriumchlorid, Natriumcitrat, Zitronensäure und Tween 80) werden in einen 1 Liter-Erlenmeyerkolben eingewogen und in Wasser unter Rühren gelöst, danach wird das Lecithin (Phospholipon G 90), Dexpanthenol, Tocopherolacetat und der Wirkstoff (Cyclosporin A) zugesetzt und unter Rühren dispergiert. Anschließend wird die Mischung ca. 10 min im Ultraturax homogenisiert und in einen Hochdruckhomogenisator überführt. Bei ca. 1500 bar wird diese Mischung solange homogenisert bis man eine kolloidale Zubereitung erhält, deren Tröpfchen- bzw. Partikelgröße in einem Malvem Zetasizer einen Druchmesser < 100 nm und einen Polydispersitätsindex < 0.4 aufweist. Die kolloidale Zubereitung wird danach in einer Sterilbank sterilfiltriert und je 20 ml werden in vorsterilisierte Braunglasfläschchen abgefüllt und anschließend mit einer Pumpsprayaufsatz, der eine sterile Mehrfachentnahme ermöglicht, verschlossen.

### Beispiel 2: Kolloidale Lösung zur Inhalation

Eine kolloidale Zubereitung aus den in der nachfolgenden Tabelle angegebenen Bestandteilen wird wie oben beschrieben hergestellt und nach Sterilfiltration werden jeweils 4 ml in sterile 6 ml Braunglasfläschchen abgefüllt und verschlossen. Der Inhalt wird dann bei Bedarf in das Medikamentenreservoir eines elektronischen Verneblers, wie z.B. den eFlow der Fa. PARI, überführt und das entstehende Aerosol kann dann inhaliert werden, um z.B. Abstossungreaktionen nach Lungentransplantationen oder die Ausbildung einer Bronchiolitis obliterans zu vermeiden.

| | **Konzentration [w/w%]** | **Funktion** |
|---|---|---|
| Cyclosporin A | 0.50 | Wirkstoff |
| Tween 80 | 0.35 | Stabilisator |
| Lipoid S100 | 4.5 | Träger |
| NaCl | 0.85 | Isotonisierungsmittel |
| Dinatrium-Edetat | 0.05 | Komplexierungsmittel |
| Wasser für Injektionszwecke | ad 100.0 | Lösungsmittel |

Der pH-Wert der klaren, schwach opaleszenten liposomalen Lösung bei 20 °C betrug 4.5, die Osmalität 0.32 osmollkg. Die dynamische Viskosität wurde mit 1.35 mPas ermittelt, die Oberflächenspannung betrug 36 mN/m.

Die kolloidale Lösung wurde mit einem speziell angepassten Schwingmembranvernebler vom Typ PARI eFlow aerosolisiert und das Aerosol mit einem PARI Atemzugsimulator charakterisiert. Dabei wurden zwei unterschiedliche Inhalationsmanöver getestet, nämlich das eines Kindes (16 Aternzüge pro Minute mit einem Volumen von je 225 ml, Verhältnis Inhalation zu Exhalation von 40 : 60) und das eines Erwachsenen (15 Atemzüge pro Minute mit einem Volumen von je 500 ml, Verhältnis Inhalation zu Exhalation von 1 : 1). Die Aerosolcharakteristika sind in der folgenden Tabelle aufgeführt:

| | **Erwachsen-Atemmuster (15 Atemzüge a 500 ml, Inhalation : Exhalation = 50 : 50)** | | **Kinder-Atemmuster (16 Atemzüge a 225 ml, Inhalation : Exhalation = 40 : 60)** | |
|---|---|---|---|---|
| | Mittelwert | Standardabweichung | Mittelwert | Standardabweichung |
| **Aerosol Dosis vom Mundstück [µg]** | 9577,1 | 745,1 | 8954, 4 | 1085,8 |
| **Vernebler Rückstand [µg]** | 1606,8 | 559,2 | 2123.9 | 352,8 |
| **Aerosol Verluste [µg]** | 2872.5 | 255,9 | 2763,3 | 376,2 |
| **Verneblungszeit [min]** | 9,5 | 0,6 | 11,6 | 1,4 |
| **Aerosol Dosis [% Einge-füllte Dosis]** | 66,5 | 4,5 | 62,9 | 8,4 |
| **Vernebler Rückstand [% Eingefüllte Dosis]** | 11,2 | 3,9 | 15,0 | 2,5 |
| **Aerosol Verluste [%]** | 20,0 | 2,0 | 19,4 | 2,5 |
| **Wiederfindung [% Einge-füllte Dosis]** | 97.7 | 5,0 | 97,3 | 7,1 |

Von der gleichen Inhalationslösung wurden per Laserdiffraktionsmessung zudem bei unterschiedlichen Aerosolausbringungsraten die für eine pulmonale Verabreichung relevanten Teilchengrüßenfraktionen bestimmt (siehe nachfolgende Tabelle). Es zeigte sich eine besonders hohe Effizienz der Aerosolabgabe und simulierten Deposition, die eine sehr kurze Inhalationszeit erlaubt und in hohem Maße von dem jeweiligen Inhalationsmanöver unabhängig ist.

| | **15 L/min** | | **20 L/min** | | **28.3 L/min** | |
|---|---|---|---|---|---|---|
| | **Mittel- wert** | **Std. Abw.** | **Mittel- wert** | **Std. Abw.** | **Mittel- wert** | **Std. Abw.** |
| **Mittlerer Massen Durchmesser [µm]** | 2,8 | 0,1 | 2,9 | 0,1 | 2,7 | 0,1 |
| **Geometrische Std. Abw.[ ]** | 1,5 | 0,0 | 1,5 | 0,0 | 1,5 | 0,0 |
| **Respirable Fraktion [%<3.3µ]** | 63,5 | 1,7 | 61,5 | 4,1 | 66,0 | 3,4 |
| **Respirable Fraktion [%<5µ]** | 89,4 | 1,1 | 89,2 | 2,1 | 91,2 | 1,6 |
| **Aerosol Abgabe Rate [mg/min]** | 289,0 | 10,8 | 258,3 | 40,3 | 282,5 | 28,0 |

Der Effekt der in Beispiel 12 beschriebenen Inhalationslösung auf Calu-3-Zellen (konfluente Monolayer) wurde untersucht. Dazu wurden Calu-3-Zellen (HTB- 55, ATCC, Manassas, VA, USA) in Minimum Essential Medium (MEM) mit Earl's Salts ergänzt durch L-Glutamin (PAA Laboratories GmbH, Pasching, Austria), 10 % fötales Rinderserum, 1 % nichtessenzielle Aminosäurelösung und 55 mg/500 ml Natriumpyruvat kultiviert. Die Bildung von konfluenten Monolayem mit Tight Junctions wurde durch Messung des transepithelialen elektrischen Widerstands (TEER) mit einem geeigneten Voltmeter (EVOM, World Precision Instruments, Berlin, Germany) und einer STX-2 Elektrode bestätigt. Das Alter der Zell-Monolayer betrug bei den Untersuchungen mit der Cyclosporinlösung etwa 14 Tage. Zunächst wurde das Kulturmedium entfernt, die Monolayer gewaschen und mit Ringer-Hydrogencarbonat-Pufferlösung für 20-30 Minuten vorinkubiert. Danach wurde die liposomale Lösung mittels einer Pipette zugegeben.

Der TEER wurde sofort nach der Applikation und 120 min später gemessen. Anschließend wurden die Zellen wieder gewaschen und über 24 Stunden mit Kulturmedium inkubiert. Anschließend wurde erneut der TEER gemessen. Als Vergleich wurden Ringer-Hydrogencarbonat-Pufferlösung (KRB), Propylenglykol, eine Cyclosporin-A-Lösung in Propylenglykol (62.5 mg/ml) und eine wässrige Natrium-dodecylsulfatlösung (SDS) (0.1%)verwendet. Die nachfolgende Tabelle zeigt die gefundenen TEER-Werte in Prozent der Initialwerte nach Zugabe der Ringer-Hydrogencarbonat-Pufferlösung.

| **TEER [% of 0 hours]** | | | | |
|---|---|---|---|---|
| **Konz. [%, w/v]** | **Zeit [h]** | **Mittelwert (n=3)** | **SD** | **RSD** |
| Positivkontrolle (0,1 % SDS) | **Medium** | 100 | | |
| | **KRB** | 107 | 2 | 2 |
| | **0** | 20 | 1 | 4 |
| | **1** | 19 | 1 | 7 |
| | **2** | 19 | 5 | 26 |
| | **24** | 11 | 1 | 7 |
| Negativkontrolle (KRB) | **Medium** | 100 | | |
| | **KRB** | 108 | 2 | 2 |
| | **0** | 101 | 6 | 5 |
| | **1** | 86 | 2 | 2 |
| | **2** | 83 | 0 | 0 |
| | **24** | 70 | 3 | 4 |
| Ciclosporin Liposomen 5 mg/ml | **Medium** | 100 | | |
| | **KRB** | 115 | 1 | 1 |
| | **0** | 114 | 5 | 4 |
| | **1** | 95 | 4 | 4 |
| | **2** | 90 | 1 | 1 |
| | **24** | 92 | 2 | 2 |
| Ciclosporin Liposomen 1:5 Verdünnung in KRB | **Medium** | 100 | | |
| | **KRB** | 102 | 8 | 8 |
| | **0** | 88 | 8 | 10 |
| | **1** | 99 | 11 | 12 |
| | **2** | 108 | 13 | 12 |
| | **24** | 89 | 6 | 7 |
| Ciclosporin Liposomen 1:10 Verdünnung in KRB | **Medium** | 100 | | |
| | **KRB** | 119 | 7 | -25 |
| | **0** | 114 | 7 | -25 |
| | **1** | 143 | 7 | -25 |
| | **2** | 155 | 7 | -25 |
| | **24** | 122 | 7 | -25 |
| Ciclosporin Liposomen 1:15 Verdünnung in KRB | **Medium** | 100 | | |
| | **KRB** | 123 | 3 | 3 |
| | **0** | 119 | 7 | 6 |
| | **1** | 120 | 3 | 2 |
| | **2** | 135 | 4 | 3 |
| | **24** | 101 | 3 | 3 |
| Placebo Liposomen Konzentrat. | **Medium** | 100 | | |
| | **KRB** | 123 | 3 | 3 |
| | **0** | 124 | 5 | 4 |
| | **1** | 101 | 3 | 3 |
| | **2** | 102 | 4 | 4 |
| | **24** | 102 | 2 | 2 |
| Placebo Liposomen Konzentrat Verdünnung 1:5 | **Medium** | 100 | | |
| | **KRB** | 111 | 5 | 5 |
| | **0** | 97 | 3 | 3 |
| | **1** | 96 | 5 | 5 |
| | **2** | 97 | 3 | 3 |
| | **24** | 97 | 6 | 6 |
| Placebo Liposomen Konzentratverdünnung1:10 | **Medium** | 100 | | |
| | **KRB** | 124 | 3 | 3 |
| | **0** | 102 | 3 | 3 |
| | **1** | 99 | 2 | 2 |
| | **2** | 119 | 1 | 1 |
| | **24** | 84 | 5 | 6 |
| Placebo Lipo-somen Konten-tratverdünnung 1:15 | **Medium** | 100 | | |
| | **KRB** | 132 | 2 | 1 |
| | **0** | 115 | 14 | 12 |
| | **1** | 110 | 7 | 6 |
| | **2** | 121 | 9 | 7 |
| | **24** | 80 | 1 | 1 |

| | | **TEER nach 2 h** | | **TEER nach 24 h** | |
|---|---|---|---|---|---|
| **Testzubereitungen** | **Konz. [%]** | **Mittelwert [%]** | **SD** | **Mittelwert [%]** | **SD** |
| KRB | | 134 | 19.87 | 89 | 18.35 |
| CSA / Propylenglycol | 11.050 | 9 | 2.46 | 22 | 4.65 |
| Propylenglycol | 10.000 | 14 | 2.42 | 43 | 2.71 |
| | 5.000 | 36 | 8.75 | 75 | 10.36 |
| | 0.500 | 120 | 12.82 | 83 | 8.77 |
| | 0.100 | 116 | 3.80 | 83 | 3.90 |
| SDS (Sodium Dodecyl Sulfate) | 0.100 | 3 | 1.93 | 2 | 1.25 |
| | 0.010 | 7 | 0.80 | 2 | 0.37 |
| | 0.001 | 114 | 20.30 | 107 | 16.29 |

Die gemessenen TEER-Werte zeigen, dass die erfindungsgemäße Zusammensetzung keinen oder nur einen sehr geringen und überwiegend reversiblen Einfluss auf die Integrität der Calu-3-Monolayer hat. Dagegen bewirken Natriumdodecylsulfat (SDS), synonym Natriumlaurylsulfat, Propylenglykol und Cyclosporin A gelöst in Propylenglykol erhebliche und überwiegend nichtreversible Schädigungen des Calu-3-Zellmonolayer. Daraus kann unter anderem geschlossen werden, dass Propylenglykol kein geeigneter Träger für Cyclosporin A für die inhalative Anwendung sein dürfte.

### Beispiel 3: Kolloidale Lösung

Die folgenden Rezepturen A und B (siehe nachstehende Tabellen) wurden analog Beispiel 1 hergestellt und steril in Braunglasfläschchen abgefüllt.

| **Beispiel 3, Rezeptur A** | **Konzentration (Gew.-%)** |
|---|---|
| Cyclosporin A | 0,50 |
| Tween 80 | 0,35 |
| Lipoid S100 | 4,50 |
| Natriumdihydrogenphosphat monohydrat | 0,215 |
| Natriumhydrogenphosphat dodecahydrat | 0,34 |
| Natriumchlorid | 0,80 |
| Dinatrium-Edetat | 0,02 |
| Wasser für Injektionszwecke | ad 100,0 |

| **Beispiel 3, Rezeptur B** | **Konzentration (Gew.-%)** |
|---|---|
| Cyclosporin A | 0,40 |
| Tween 80 | 0,28 |
| Lipoid S 100 | 3,60 |
| Natriumdihydrogenphosphat monohydrat | 0,215 |
| Natriumhydrogenphosphat dodecahydrat | 0,34 |
| Natriumchlorid | 0,80 |
| Dinatrium-Edetat | 0,02 |
| Wasser für Injektionszwecke | ad 100,0 |

Die kolloidalen Lösungen sind insbesondere für die Inhalation geeignet. Darüber hinaus können sie auch für topische und ophthalmische Anwendungen eingesetzt werden.

### Beispiel 4: Kolloidale Lösung

Die nachfolgende Rezeptur (siehe nachstehende Tabelle) wurde analog Beispiel 1 herstellt und steril abgefüllt.

| **Beispiel 4, Rezeptur** | **Konzentration (Gew.-%)** |
|---|---|
| Cyclosporin A | 0,50 |
| Tween 80 | 0,35 |
| Lipoid S100 | 4,50 |
| Vitamin E TPGS | 0,35 |
| Natriumdihydrogenphosphat monohydrat | 0,25 |
| Natriumhydrogenphosphat dodecahydrat | 0,25 |
| Natriumchlorid | 0,85 |
| Dinatrium-Edetat | 0,02 |
| Wasser für Injektionszwecke | ad 100,0 |

Die leicht opaleszierende Lösung wurde anschließend charakterisiert; die Ergebnisse finden sich in der nachstehenden Tabelle.

| **Parameter** | **Messwert** |
|---|---|
| pH | 6,51 |
| Dynamische Viskosität | 1,36 mPas*s |
| Oberflächenspannung | 32,8 mN/m |
| Refraktionsindex | 1,342 |
| Osmolalität | 372 mOsmol/kg |
| Dichte | 1,007 g/cm³ |
| Medianwert Liposomendurchmesser | 35,7 nm |
| Polydispersitätsindex | 0,21 |

Des Weiteren wurde ein tatsächlicher Gehalt an CsA von 4,83 mg/ml gemessen. Der Gehalt an Verunreinigungen betrug 0,81 mg/ml.

Nach dreimonatiger Lagerung bei 5 °C zeigten sich alle gemessenen Parameter nur unwesentlich verändert, darunter z. B. der CsA-Gehalt (4,93 mg/ml), der Gehalt an Verunreinigungen (0,36 mg/ml) sowie der mediane Liposomendurchmesser (36,6 nm). Nach dreimonatiger Lagerung bei 25 °C und 60 % rh zeigte sich die Zubereitung ebenfalls ausgesprochen stabil; insbesondere der Gehalt an CsA und Verunreinigungen blieben weitgehend konstant. Der mediane Liposomendurchmesser war leicht auf 44,4 nm erhöht, was jedoch keinen Einfluss auf die Performance der Zubereitung haben sollte.
3,2 ml (entsprechend 15 mg CsA) der kolloidalen Lösung wurde mit einem speziell angepassten elektronischen Schwingmembranvernebler vom Typ PARI eFlow 30 L mit einer Mischkammer und Ein-/Ausatemventilen aerosolisiert und die Tröpfchengrößenverteilung des derart erzeugten Aerosols per Laserdiffraktion mit Hilfe eines Malvem MasterSizerX bei einer Flussrate von 20 l/min charakterisiert. Dabei wurde ein massenmittlerer Partikeldurchmesser von 2,8 µm bei einer geometrischen Standardabweichung von 1,5 gemessen. Die Partikelfraktion < 5 µm ("respirable fraction") betrug 89,4 %, die Fraktion mit einer Partikelgröße < 3,3 µm betrug 63,5 %. Die Ausbringungsrate ("total output rate") belief sich auf 289 mg/ml.

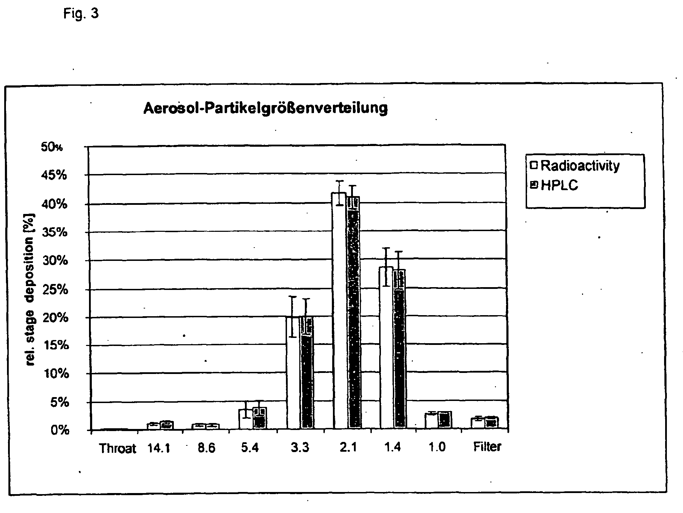

Darüber hinaus wurde das Aerosol in einem Atemsimulationstest mit einem PARI A-temzugsimulator charakterisiert, wobei das typische Inhalationsmuster eines Erwachesenen (siehe Beispiel 2) eingestellt wurde, sowie durch einen Kaskadenimpaktor vom Typ Next Generation Impactor bei einer Flussrate von 15 l/min, einer Temperatur von 23 °C und einer relativen Feuchte von 50 %. Dabei wurden die Aerosolcharakteristika erhalten, die in der nachstehenden Tabelle aufgeführt sind. Die Inhalationszeit betrug 11 Minuten. Die Berechnung der jeweiligen CsA-Fraktionen erfolgte nach Bestimmung des Wirkstoffs über eine HPLC-Gehaltsbestimmung. In einem weiteren Test wurde die Inhalationslösung mit ⁹⁹mTc-DTPA radiomarkiert, derselben Versuchsanordnung unterworfen und die Aerosolcharakteristika über die jeweilige Radioaktivität der deponierten Fraktionen ermittelt. Die Messergebnisse beider Versuche stimmten weitestgehend überein. Figur 3 zeigt die Verteilung der Aerosolfraktionen auf den jeweiligen Impaktorstufen (basierend auf Radioaktivitätsmessung und HPLC), wobei die Werte auf der Abszisse die Einheit µm besitzen.

| **Parameter** | **mg** | **%** |
|---|---|---|
| Füllmenge CsA | 15,0 | 100 |
| Ausgebrachte Dosis CsA ("delivered dose") | 11,4 | 75,9 |
| Im Inhalator zurückgebliebenes CsA | 1,3 | 8,5 |
| Aerosolverlust CsA | 2,0 | 13,1 |

Mit der Zubereitung wurde ferner eine Gammaszintigraphie-Studie zur Bestimmung der Lungendeposition von CsA in vivo durchgeführt. Dabei wurden 12 Patienten mit Lungentransplantaten (7 mit beiseitigen [DLTx] und 5 mit einseitigen [SLTx] Transplantaten) mit 2 ml (ca. 10 mg CsA) der Inhalationslösung behandelt. Jede verabreichte Dosis der Inhalationslösung wurde zuvor mit 4 MBq 88mTc-DTPA (Pentacis, CIS-Diagnostik) radiomarkiert und anschließend in einen elektronischen Schwingmembranvernebler vom Typ PARI eFlow 30L gefüllt. Die Patienten wurden instruiert, langsam und tief zu inhalieren. Jeweils nachdem das Reservoir des Verneblers geleert war, wurden von den Patienten die Kopf- und Brustregionen mit einer Gamma-Camera vom Typ Siemens Diacam aufgenommen.

Figur 1 zeigt ein typisches Bild eines der behandelten Patienten mit beidseitigem Lungentransplantat, Figur 2 das eines Patienten mit einseitigem Transplantat. Die Aufnahmen verdeutlichen die gleichmäßige Deposition des feinen Aerosols in der Lunge. Die Inhalation wurde von allen Patienten gut vertragen; es war keine Prämedikation notwendig, wie sie nach Literaturangaben bei der Inhalation einer bekannten CsA-Zubereitung notwendig war, in welcher der Wirkstoff in Propylenglykol gelöst vorgelegen hatte.

In einem weiteren Versuch mit denselben Patienten wurde eine Dosis von 4 ml bei unveränderter Wirkstoffkonzentration verabreicht. Auch diese Dosis wurde ohne Prämedikation vertragen.

Die quantitative Bestimmung der Radioaktivität zeigte eine durchschnittliche Lungendeposition von 36 % der in den Vernebler eingefüllten CsA-Dosis. Die relative Deposition in der Lunge war im Falle der niedrigeren Dosis von 2 ml etwas höher als bei der höheren Dosis von 4 ml. Die nachstehende Tabelle fasst die Resultate zusammen.

| **Depositionsort** | **Mittelwert (alle)** | **2ml** | **4ml** | **DLTx** | **SLTx** |
|---|---|---|---|---|---|
| Intrapulmonal | 36.0% | 39.8% | 32.6% | 37.0% | 36.0% |
| *(davon peripher)* | *19.0%* | *20.4%* | *17.2%* | *20.0%* | *18.0%* |
| Extrathorakal | 15.0% | 16.3% | 13.6% | 14.0% | 15.0% |
| Rückstand im Vernebler | 18.0% | 10.1% | 26.4% | 19.0% | 18.0% |
| Aerosolverlust/exhaliert | 31.0% | 33.9% | 27.4% | 31.0% | 31.0% |
| Inhalationszeit | | 9.0 min | 19.1 min | | |

Des Weiteren wurden die pharmakokinetischen Parameter bei inhalativer Verabreichung von 10 mg bzw. 20 mg CsA durch Blutuntersuchungen bestimmt. Die dabei erhaltenen Ergebnisse sind in der folgenden Tabelle angegeben:

| | | | |
|---|---|---|---|
| | 10 mg | 20 mg | |
| T_{1/2} | 5.2 h | 6.1 h | p = 0.034 |
| AUC | 110 h · ng/ml | 190 h · ng/ml | p = 0.025 |
| cₘₐₓ | 20 ng/ml | 31 ng/ml | |
| MRT (mean residence time) | 7.8 h | 8.6 h | no sig. |

Überraschenderweise wurde gefunden, dass die liposomale CsA-Inhalationslösung nach Inhalation nur in geringem Anteil ins Blut übergeht, insbesondere eine sehr kurze Halbwertszeit von 5.2 bzw. 6.1 Stunden aufweist.

Bisher veröffentlichte Daten (Corcoran TE et al.: Preservation of post-transplant lung function with aerosol cyclosporin. Eur. Respir. J., 23(3), 378-83 (2004)) sprechen von einer Halbwertszeit von ca. 40 Stunden nach inhalativer Gabe von 300 mg CsA / Propylenglykol Inhalationslösung. Der Vorteil der kurzen Halbwertszeit liegt in der Möglichkeit einer häufigeren Verabreichung, z.B. einer ein- oder zweimalige Gabe pro Tag. Dadurch werden gleichmäßigere Therapiespiegel auch in der Lunge zu erzielen sein und eine zuverlässigere Therapie resultieren.

Darüber hinaus hat sich eine Korrelation zwischen der peripher deponierten CsA-Dosis und den pharmakologischen Parametern AUC (Area under the curve) und cₘₐₓ (maximale Konzentration) für die liposomale CsA-Therapie ergeben: Dadurch kann der CsA-Wirkspiegel im Zielareal des Zielorgens, d.h. in der Peripherie der Lunge, durch einfache Messung der Blutspiegel für jeden Patienten abgeschätzt werden. Dies ermöglicht ein einfaches Monitoring der Therapie.

### Beispiel 5: Kolloidale Lösung zum Einträufeln ins Auge

Ein kolloidale Zubereitung aus den in der nachfolgenden Tabelle angegebenen Bestandteilen wird wie oben beschrieben hergestellt und nach Sterilfiltration werden jeweils 0.25 ml aseptisch unter einem Laminar air flow in sterile birnenförmige 0.5 ml Polyethylen-blow fill seal vials (PE), die mit sterilem Stickstoff geformt werden, abgefüllt und danach in Aluminiumblister, die mit Stickstoff begast sind, eingeschweißt. Die kolloidale Lösung kann aus diesen sterilen unit dose vials, die eine tropfenförmige Produktentnahme erlauben, ins Auge geträufelt werden, um z.B. ein Abstosssungsreaktion nach einer Homhauttransplantation oder andere Entzündungsprozesse zu behandeln.

| | **Konzentration [w/w%]** | **Funktion** |
|---|---|---|
| Cyclosporin A | 0.50 | Wirkstoff |
| Tobramycin | 1.0 | Wirkstoff |
| Tween 80 | 0.35 | Stabilisator |
| Lipoid S100 | 4.50 | Träger |
| NaCl | 0.56 | Isotonisierungsmittel |
| KH₂PO₄ | 0.68 | Puffersubstanz |
| Natriumhydroxid | q.s. | Puffersubstanz |
| Dinatrium-Edetat | 0.05 | Komplexierungsmittel |
| Wasser für Injektionszwecke | ad 100.0 | Lösungsmittel |

### Beispiel 6:

Jeweils 0.2 g Cyclosporin A und Tacrolimus werden in einer liposomalen isotonischen Placebolösung, die Lecithin und Polysorbat 80 im Gewichts-Verhältnis 10: 1 in einer isotonischen Kochsalzlösung enthält, mit Hilfe eines Ultraturax dispergiert und danach in einem Microfluidizer bei 1500 bar in mehreren Zyklen so hochdruckhomogenisiert, daß in einem Malvem Zetasizer eine kolloidale Zubereitung mit einem Durchmesser kleiner 80 nm und einem Polydispersitätsindex < 0.35 erhalten wird. Nach Sterilfiltration werden jeweils 2 ml aseptisch unter einem Laminar air flow in sterile Polyethylen-blow fill seal vials (PE), die mit sterilem Stickstoff geformt werden, abgefüllt und danach in Aluminiumblister, die mit Stickstoff begast sind, eingeschweißt. Die kolloidale Lösung kann aus diesen sterilen unit dose vials, die eine tropfenförmige Produktentnahme erlauben, für die pulmonale, nasale oder topische Applikation verwendet werden, um unerwünschte Autoimmun-Erkrankungen zu behandeln.

### Beispiel 7: Liposomale Lösung zur Inhalation oder zur Anwendung an Auge und Ohr

In eine isotonische, mittels Hochdruckhomogenisation hergestellte, kolloiddisperse Placebolösung bestehend aus 10% Lipoid S 100, 0.7% Polysorbat 80, 0.8% Natriumchlorid und jeweils 0.01% Natrium-EDTA und Tocopherol-acetat werden 0.4% Ciclosporin A und 0.04% Budesonid dispergiert und kolloiddispers so eingearbeitet, daß eine liposomale Zubereitung mit einem Durchmesser < 75 nm und einem Polydispersitätsindex < 0.3 erhalten wird. In einem Transmissionselektronenmikroskop sieht man sphärische unilamellare liposomale Strukturen von 55-75 nm, die gut mit Ergebnissen von 40 -55 nm übereinstimmen, die mittels Photonen- Korrelations-spektroskopie (PCS) ermittelt wurden. Nach Sterilfiltation werden für die Augen- und Ohrenbehandlung jeweils 0.25ml und für die Verwendung in Verneblern je 2 ml in Polypropylen vials, die mit Stickstoff begast sind, abgefüllt und zwecks besserer Haltbarkeit einzeln in stickstoffbegaste Aluminiumblister eingeschweißt. Nach Vernebelung mit dem elektronischne Vernebler eFlow wird das Produkt zur Behandlung von Lungenerkrankungen wie z.B. Asthma und COPD inhaliert.

### Beispiel 8:

In eine isotonische, mittels Hochdruckhomogenisation hergestellte, kolloiddisperse Placebolösung bestehend aus 40% Lipoid S 100, und jeweils 2% Polysorbat 80, und Vitamin E-TPGS, 0.8% Natriumchlorid und 0.02% Natrium-EDTA werden jeweils 1.5% Ciclosporin A und Sirolimus (Rapamycin) dispergiert und kolloiddispers so eingearbeitet, daß eine liposomale Zubereitung mit einem Durchmesser < 100 nm und einem Polydispersitätsindex < 0.3 erhalten wird. Nach Sterilfiltation werden jeweils 2 ml in Polyethylen vials, die mit Stickstoff begast sind, abgefüllt und zwecks besserer Haltbarkeit einzeln in stickstoffbegaste Aluminiumblister eingeschweißt. Das Produkt wird inhalativ zur Behandlung von interstitiellen Lungenerkrankungen, wie Sarkoidose und pulmonare Fibrose, eingesetzt. Alternativ kann es auch zurm Eintäufeln in das Auge nach Homhauttransplantationen eingesetzt werden.

### Beispiel 9: Kolloidale Lösung zur topischen Behandlung von Haut, Auge und Ohr

| | **Konzentration [w/w%]** | **Funktion** |
|---|---|---|
| Cyclosporin A | 0.50 | Wirkstoff |
| Tween 80 | 0.35 | Stabilisator |
| Lipoid S100 | 4.50 | Löslichkeitsverbesserer |
| NaCl | 0.5 | Isotonisierungsmittel |
| Dexpanthenol | 5.0 | Hautschutzstoff |
| Dinatrium-Edetat | 0.05 | Komplexierungsmittel |
| Wasser für Injektionszwecke | ad 100.0 | Lösungsmittel |

Die in der voranstehenden Tabelle angegebenen wasserlöslichen Hilfsstoffe werden in einen 1 liter Erlenmeyerkolben eingewogen und in Wasser unter Rühren gelöst, danach wird das Lecithin (Lipoid S 100 oder auch Phospholipon G 90) und der Wirkstoff (Cyclosporin) zugesetzt und unter Rühren dispergiert. Anschließend wird die Mischung ca. 10 min im Ultraturax homogenisiert und in einen Hochdruckhomogenisator überführt. Bei ca. 1500 bar wird diese Mischung solange homogenisert bis man eine kolloidale Zubereitung erhält, deren Tröpfchen- bzw. Partikelgröße in einem Malvem Zetasizer einen Druchmesser < 100 nm und einen Polydispersitätsindex < 0.4 aufweist. Die kolloidale Zubereitung wird danach in einer Sterilbank sterilfiltriert und in vorsterilisierte Braun-Glasfläschchen mit Volumina von 5 - 50 ml abgefüllt und anschließend mit einem sterilen Pumpdosieraufsatz, der eine tropfenförmige sterile Mehrfachentnahme ermöglicht, verschlossen.

### Beispiel 10:

In eine isotonische, mittels Hochdruckhomogenisation hergestellte, kolloiddisperse Placebolösung bestehend aus 40% Phospholipon und jeweils 2% Polysorbat 80, und Vitamin E-TPGS, 0.8% Natriumchlorid und 0.02% Natrium-EDTA werden jeweils 3% Dexpanthenol und danach je 1% Ciclosporin A, Amphotericin B dispergiert und kolloiddispers so eingearbeitet, daß eine liposomale Zubereitung mit einem Durchmesser < 100 nm und einem Polydispersitätsindex < 0.3 erhalten wird. Nach Sterilfiltation werden jeweils 2 ml in Polyethylen vials, die mit Stickstoff begast sind, abgefüllt und zwecks besserer Haltbarkeit einzeln in stickstoffbegaste Aluminiumblister eingeschweißt. Das Produkt wird inhalativ zur Prävention und Behandlung von möglichen Abstossungsreaktioinen nach Transplantation von Organen oder Organteilen wie Lunge, Nase, Haut, Hornhaut, Ohr und sich daraus ergebenden Folgeerkrankungen eingesetzt.

### Beispiel 11:

In eine isotonische, mittels Hochdruckhomogenisation hergestellte, kolloiddisperse Placebolösung bestehend aus 20% Phospholipon und jeweils 1% Polysorbat 80, und Vitamin E-TPGS, 0.8% Natriumchlorid und 0:02% Natrium-EDTA werden je 2% Hyaluronsäure sowie Natriumcromoglycat und 1 % Ciclosporin A dispergiert und kolloiddispers so eingearbeitet, daß eine kolloiddisperse Zubereitung mit einem Durchmesser < 100 nm und einem Polydispersitätsindex < 0.3 erhalten wird. Nach Sterilfiltation werden jeweils 2 ml in Polyethylen vials, die mit Stickstoff begast sind, abgefüllt und zwecks besserer Haltbarkeit einzeln in stickstoffbegaste Aluminiumblister eingeschweißt. Das Produkt wird inhalativ zur Prävention und Behandlung von chronisch obstruktiver Bronchitis, parenchymalen, fibrotischen und interstitiellen Lungenerkrankungen bzw. -entzündungen, sowie topisch zur Unterdrückung von autoimmunbedingten Erkrankungen und Wundheilung von Haut, Nase und Ohr eingesetzt

### Beispiel 12:

Analog Beispiel 2 wurde eine liposomale Inhalationslösung enthaltend Cyclosporin A (4.5 mg/ml), Phospholipid (Lipoid S100, 40 mg/ml), Polysorbat 80 (Tween 80, mg/ml), Natriumchlorid (8.5 mg/ml) und Dinatrium-Edetat (0.5 mg/ml) hergestellt. Die Homogenisation erfolgte mit einem Hochdruckhomogenisator bei 1500 bar und 2 Zyklen. Die mittlere Teilchengröße der Liposomen betrug etwa 50 nm (gemessen als z-Average) bei einem Polydispersitätsindex von etwa 0.25. Die Liposomen (jeweils 2 ml) wurden nach Sterilfiltration unter aseptischen Bedingungen in speziell ausgeformete 3 ml vials abgefüllt, die formschlüssig in einen Dreh-Hubdeckel eines eFlows eingepaßt werden können. Beim Verschließen der Medikamentenaufnahmevorrichtung wird die Membran des PE-vials durchstoßen, so daß der Inhalt ohne ein manuelles einfüllen in den Verenbeler inhaliert werden kann.

### Beispiel 13:

Die liposomale Formulierung kann auch mit wasserlöslichen Wirkstoffen kombiniert werden, wie an den nachfolgend aufgeführten Beispielen ersichtlich ist. Die wasserlöslichen Hilfsstoffe werden gemäß der prozentualen Gewichts-Zusammensetzung (G/G) wie in den untenstehenden Tabellen aufgerührt in einen 200 Liter Ansatzkessel eingewogen und in Wasser für Injektionszwecke unter Rühren gelöst. Darin werden dann die wasserlöslichen Wirkstoffe, wie z.B. Heparin-Natrium (Rezeptur A) oder Salbutamolsulfat (Rezeptur B) gelöst, Lecithin und das lipophile Cyclosporin A zugesetzt und unter Rühren dispergiert, danach die Mischung ca. 10 min im Ultraturax homogenisiert und in einen Hochdruckhomogenisator überführt. Bei ca. 1000 bar wird diese Mischung in 5 Zyklen hochdruckhomogenisert. Danach wird ein Muster gezogen und gemäß der inprozess Kontrolle die Spezifikation überprüft. Ist die Tröpfchen- bzw. Partikelgröße, gemessen in einem Malvern Zetasizer < 100 nm und weist diese einen Polydispersitätsindex < 0.35 auf, wird die kolloidale Zubereitung sterilfiltriert. Jeweils 2 ml werden in einem aseptischen Abfüllprozess in einem Sterilraum gemäß einem blow fill Verfahren in Polyethylen-Ampullen abgefüllt und zu je 5 Stück in stickstoffbegaste Aluminumblisterfolien eingeschweisst.

| **Beispiel 13, Rezeptur A** | **Konzentration [G/G%]** |
|---|---|
| Cyclosporin A | 0.40 |
| Tween 80 | 0.28 |
| Phospholipon G 90 | 3.60 |
| NaCl | 0.5 |
| Heparin-Natrium | 2.0 |
| Natriumedetat | 0.025 |
| Wasser für injektion | ad 100.0 |

| **Beispiel 13, Rezeptur B** | **Konzentration [G/G%]** |
|---|---|
| Cyclosporin A | 0.40 |
| Tween 80 | 0.28 |
| Lipoid S 100 | 3.60 |
| NaCl | 0.5 |
| Salbutamol Sulfat | 0.50 |
| Natrumedeteat | 0.05 |
| Wasser für Injektion | ad 100.0 |

## Patentansprüche

1. Flüssige pharmazeutische Zusammensetzung, enthaltend:
(a) eine therapeutisch wirksame Dosis eines Cyclosporin;
(b) eine wässrige Trägerflüssigkeit;
(c) eine erste löslichkeitsverbessernde Substanz, ausgewählt aus der Gruppe der Phospholipide, und
(d) eine zweite löslichkeitsverbessernde Substanz, ausgewählt aus der Gruppe der nichtionischen Tenside,
wobei das Phospholipid eine Mischung natürlicher Phospholipide ist und wobei die Zusammensetzung das Cyclosporin in liposomal solubilisierter Form enthält.

2. Zusammensetzung nach Anspruch 1, **dadurch gekennzeichnet, dass** es sich bei dem Cyclosporin um Cyclosporin A handelt.

3. Zusammensetzung nach Anspruch 2, **dadurch gekennzeichnet, dass** sie einen Gehalt an Cyclosporin A von etwa 0.2 bis etwa 20 mg/mL aufweist.

4. Zusammensetzung nach einem der voranstehenden Ansprüche, **dadurch gekennzeichnet, dass** das Phospholipid ein Lecithin mit einem Anteil ungesättigeter Fettsäuren ist, beispielsweise Lipoid S 100, Phospholipon G90 -100 oder ein vergleichbares Lecithin.

5. Zusammensetzung nach einem der voranstehenden Ansprüche, **dadurch gekennzeichnet, dass** sie einen Gehalt des Phospholipids von etwa 0.2 bis etwa 15 Gew.-% und vorzugsweise von etwa 1 bis etwa 8 Gew.-% aufweist.

6. Zusammensetzung nach einem der voranstehenden Ansprüche, **dadurch gekennzeichnet, dass** sie einen Gehalt des nichtionischen Tensids von etwa 0.01 bis etwa 5 Gew.-% aufweist, vorzugsweise von 0.1 - 2 Gew.-%.

7. Zusammensetzung nach einem der voranstehenden Ansprüche, **dadurch gekennzeichnet, dass** das nichtionische Tensid aus der Gruppe der Polysorbate ausgewählt ist, vorzugsweise Polysorbat 80 ist.

8. Zusammensetzung nach einem der voranstehenden Ansprüche, **dadurch gekennzeichnet, dass** sie Liposomen mit einem mittleren Durchmesser von maximal etwa 100 nm und/oder einem Polydispersitätsindex von maximal etwa 0.5 enthält.

9. Zusammensetzung, nach einem der voranstehenden Ansprüche, **dadurch gekennzeichnet, dass** sie steril und weitgehend frei von festen Partikeln mit einem Teilchendurchmesser von mehr als 3 µm ist, und vorzugsweise frei von festen Wirkstoffpartikeln mit einem Teilchendurchmesser von mehr als 500 nm ist.

10. Zusammensetzung nach einem der voranstehenden Ansprüche, **dadurch gekennzeichnet, dass** sie weitgehend frei von organischen Lösungsmitteln ist.

11. Verfahren zur Herstellung einer Zusammensetzung nach einem der voranstehenden Ansprüche, **gekennzeichnet durch** einen Schritt der Hochdruckhomogenisation.

12. Verwendung einer Zusammensetzung nach einem der Ansprüche 1 bis 10 zur Herstellung eines Arzneimittels zur oralen, nasalen, ophthalmischen, pulmonalen, parenteralen, topischen oder mukosalen Applikation.

13. Verwendung nach Anspruch 12, **dadurch gekennzeichnet, dass** die pulmonale oder nasale Applikation nach Überführung der Zusammensetzung in ein Aerosol erfolgt.

14. Verwendung einer Zusammensetzung nach einem der Ansprüche 1 bis 10 zur Herstellung eines Arzneimittels zur Prophylaxe oder Behandlung von Autoimmunerkrankungen, Hautkrankheiten, Erkrankungen der Sinnesorgane (Augen, Nase, Ohr), Befindlichkeitsstörungen, und Lungenerkrankungen, insbesondere Asthma, Refractory Asthma, chronisch obstruktiver Bronchitis, parenchymalen, fibrotischen und interstitiellen Lungenerkrankungen oder -entzündungen, Lungenkrebs, und vorzugsweise zur Prävention und Behandlung akuter oder chronischer Organtransplantat-Abstoßungsreaktionen nach Lungen-, Stammzell-, Knochmark-, Herz- oder einer sonstigen Organtransplantation und deren Folgeerkrankungen wie Bronchiolitis obliterans.

15. Verwendung nach einem der Ansprüche 12 bis 14, wobei die Verabreichung des Arzneimittels ohne Prämedikation erfolgt.

16. Verfahren zur Erzeugung eines Aerosols mittels Vernebler durch Vernebelung einer pharmazeutischen Zusammensetzung nach einem der Ansprüche 1 bis 10.

17. Verfahren nach Anspruch 16, **dadurch gekennzeichnet, daß** der Prozentsatz von Tröpfchen < 5 µm zwischen 50% und 98 % und besonders bevorzugt 60 - 90% beträgt und die Tröpfchenverteilung eine geometrische Standardabweichung < 2.2 und besonders bevorzugt < 1.8 aufweist.

18. Verfahren nach Anspruch 16, **dadurch gekennzeichnet, daß** der Prozentsatz von Tröpfchen < 3.5 µm zwischen 40% und 95 % beträgt und besonders bevorzugt zwischen 50 und 85% beträgt.

19. Verfahren nach Anspruch 16, dadurch gekenenzeichnet, daß die Lungendeposition mindstens 30% beträgt und das Verhältnis von zentraler zu peripherer Deposition von 30 : 70 - 70 : 30.

## Claims

1. Liquid pharmaceutical composition comprising:
(a) a therapeutically effective dose of a ciclosporin;
(b) an aqueous carrier liquid;
(c) a first solubility enhancing substance selected from the group of phospholipids and
(d) a second solubility enhancing substance selected from the group of nonionic surfactants,
wherein the phospholipid is a mixture of natural phospholipids, and wherein the composition contains the ciclosporin in a liposomally solubilised form.

2. Composition according to claim 1, wherein the ciclosporin is ciclosporin A.

3. Composition according to claim 2, wherein the content of ciclosporin A is from about 0.2 to about 20 mg/ml.

4. Composition according to any one of the preceding claims, wherein the phospholipid is a lecithin containing unsaturated fatty acids, for example, Lipoid S100, Phospholipon G90-100 or a comparable lecithin.

5. Composition according to any one of the preceding claims, wherein the content of the phospholipid is from about 0.2 to about 15 wt.-% and preferably from about 1 to about 8 wt.-%.

6. Composition according to any one of the preceding claims, wherein the content of the nonionic surfactant is from about 0.01 to about 5 wt.-%, preferably from 0.1 to 2 wt.-%.

7. Composition according to any one of the preceding claims, wherein the nonionic surfactant is selected from the group of polysorbates, preferably is polysorbate 80.

8. Composition according to any one of the preceding claims, wherein the composition contains liposomes with an average diameter of at most about 100 nm and/or a polydispersity index of at most about 0.5.

9. Composition according to any one of the preceding claims, wherein the composition is sterile and essentially free of solid particles with a particle diameter of more than 3 µm and is preferably free of solid particles of active agent having a particle diameter of more than 500 nm.

10. Composition according to any one of the preceding claims, wherein the composition is essentially free of organic solvents.

11. Method for the preparation of a composition according to any one of the preceding claims, wherein the method comprises a step of high-pressure homogenisation.

12. Use of a composition according to any one of claims 1 to 10 for the preparation of a medicament for oral, nasal, ophthalmic, pulmonary, parenteral, topical or mucosal application.

13. Use according to claim 12, wherein the pulmonary or nasal application is carried out after conversion of the composition into an aerosol.

14. Use of a composition according to any one of claims 1 to 10 for the preparation of a medicament for the prophylaxis and treatment of autoimmune diseases, skin diseases, diseases of the sensory organs (eyes, nose, ear), malaise and pulmonary diseases, in particular, asthma, refractory asthma, chronic obstructive bronchitis, parenchymal, fibrotic and interstitial lung diseases and inflammations, lung cancer, and preferably for the prevention and treatment of acute and chronic organ transplant rejection reactions after lung, stem cell, bone marrow, heart or other organ transplantations and the diseases resulting therefrom such as bronchiolitis obliterans.

15. Use according to any one of claims 12 to 14, wherein the administration of the medicament is carried out without premedication.

16. Process for generating an aerosol by means of a nebuliser by nebulising a pharmaceutical composition according to any one of claims 1 to 10.

17. Process according to claim 16, wherein the percentage of droplets of < 5 µm is between 50% and 98% and more preferably 60 - 90% and the droplet distribution has a geometric standard deviation < 2.2 and more preferably < 1.8.

18. Process according to claim 16, wherein the percentage of droplets of < 3.5 µm is between 40% and 95% and more preferably between 50 - 85%.

19. Process according to claim 16, wherein the pulmonary deposition is at least 30% and the ratio of central to peripheral deposition is from 30 : 70 - 70 : 30.

## Revendications

1. Composition pharmaceutique liquide, contenant :
(a) une dose thérapeutiquement efficace d'une cyclosporine ;
(b) un liquide porteur aqueux ;
(c) une première substance améliorant la solubilité, choisie dans le groupe des phospholipides, et
(d) une deuxième substance améliorant la solubilité choisie dans le groupe des tensioactifs non ioniques,
le phospholipide contenant un mélange de phospholipides naturels et la composition contenant la cyclosporine sous forme liposomale solubilisée.

2. Composition selon la revendication 1, **caractérisée en ce que** la cyclosporine est la cyclosporine A.

3. Composition selon la revendication 2, **caractérisée en ce qu'**elle présente une teneur en cyclosporine A d'environ 0,2 à environ 20 mg/ml.

4. Composition selon l'une des revendications précédentes, **caractérisée en ce que** le phospholipide est une lécithine présentant une proportion d'acides gras insaturés, par exemple le lipide S 100, le Phospholipon G90-100 ou une lécithine comparable.

5. Composition selon l'une des revendications précédentes, **caractérisée en ce qu'**elle présente une teneur en phospholipide d'environ 0,2 à environ 15 % en poids et de préférence d'environ 1 à environ 8 % en poids.

6. Composition selon l'une des revendications précédentes, **caractérisée en ce qu'**elle présente une teneur en tensioactif non ionique d'environ 0,01 à environ 5 % en poids, de préférence de 0,1 à 2 % en poids.

7. Composition selon l'une des revendications précédentes, **caractérisée en ce que** le tensioactif non ionique est choisi dans le groupe des polysorbates, de préférence, il est le polysorbate 80.

8. Composition selon l'une des revendications précédentes, **caractérisée en ce qu'**elle contient des liposomes présentant un diamètre moyen au maximum d'environ 100 nm et/ou un indice de polydispersité au maximum d'environ 0,5.

9. Composition selon l'une des revendications précédentes, **caractérisée en ce qu'**elle est stérile et largement dépourvue de particules solides présentant un diamètre de particule supérieur à 3 µm et de préférence, est dépourvue de particules de substance solides présentant un diamètre de particule supérieur à 500 nm.

10. Composition selon l'une des revendications précédentes, **caractérisée en ce qu'**elle est largement dépourvue de solvants organiques.

11. Procédé de production d'une composition selon l'une des revendications précédentes, **caractérisé par** une étape d'homogénéisation à haute pression.

12. Utilisation d'une composition selon l'une des revendications 1 à 10 pour la production d'un médicament pour l'administration orale, nasale, ophtalmique, pulmonaire, parentérale, topique ou muqueuse.

13. Utilisation selon la revendication 12, **caractérisée en ce que** l'administration pulmonaire ou nasale s'effectue par transformation de la composition en un aérosol.

14. Utilisation d'une composition selon l'une des revendications 1 à 10, pour la production d'un médicament pour la prophylaxie ou le traitement de maladies auto-immunes, de maladies de la peau, de maladies des organes des sens (yeux, nez, oreilles), des altérations de l'état général et des maladies ou inflammations pulmonaires, en particulier, l'asthme, l'asthme réfractaire, la bronchopathie chronique obstructive, les maladies pulmonaires parenchymateuses, fibrotiques et interstitielles, le cancer du poumon, et de préférence, pour la prévention et le traitement des réactions de rejet aiguës ou chroniques dans les greffes d'organe, après une greffe de poumon, de cellules souches, de moelle osseuse, du coeur ou d'un autre organe et les maladies qui en découlent telles que la bronchiolite oblitérante.

15. Utilisation selon l'une des revendications 12 à 14, dans laquelle l'administration du médicament s'effectue sans prémédication.

16. Procédé de génération d'un aérosol au moyen d'un nébuliseur par nébulisation d'une composition pharmaceutique selon l'une des revendications 1 à 10.

17. Procédé selon la revendication 16, **caractérisé en ce que** le pourcentage de gouttelettes < 5 µm se situe entre 50 % et 98 % et de manière particulièrement préférée se situe entre 60 et 90 % et la répartition des gouttelettes présente un écart type géométrique < 2,2 et de manière particulièrement préférée < 1,8.

18. Procédé selon la revendication 16, **caractérisé en ce que** le pourcentage de gouttelettes < 3,5 µm se situe entre 40 % et 95 % et de manière particulièrement préférée, entre 50 et 85 %.

19. Procédé selon la revendication 16, **caractérisé en ce que** le dépôt dans les poumons est d'au moins 30 % et le rapport du dépôt central ou dépôt périphérique est de 30:70 à 70:30.
